(19) Europäisches Patentamt
European Patent Office
Office européen des brevets

(11) EP 2 803 408 B1

(12) **EUROPEAN PATENT SPECIFICATION**

(45) Date of publication and mention of the grant of the patent:
**04.11.2020 Bulletin 2020/45**

(21) Application number: **14178192.2**

(22) Date of filing: **26.09.2011**

(51) Int Cl.:
*B01J 31/02* (2006.01) *B01J 31/04* (2006.01)
*C07C 29/50* (2006.01) *C07C 31/135* (2006.01)
*C07C 45/33* (2006.01) *C07C 49/303* (2006.01)
*C07C 407/00* (2006.01) *C07C 409/14* (2006.01)
*C07B 61/00* (2006.01) *B01J 31/22* (2006.01)

(54) **Method and apparatus for producing oxide of hydrocarbon compound using same**

Verfahren und Vorrichtung zur Herstellung von Oxid aus der Kohlenwasserstoffverbindung

Procédé et appareil de production d'oxyde de composé hydrocarbure

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.10.2010 JP 2010223534**
**31.08.2011 JP 2011188167**
**08.09.2011 JP 2011195542**

(43) Date of publication of application:
**19.11.2014 Bulletin 2014/47**

(62) Document number(s) of the earlier application(s) in accordance with Art. 76 EPC:
**11828975.0 / 2 623 199**

(73) Proprietor: **Ube Industries, Ltd.**
**Ube-shi, Yamaguchi 755-8633 (JP)**

(72) Inventors:
- **Kurosawa, Kazunori**
**Ube-shi, Yamaguchi 755-8633 (JP)**
- **Funatsu, Joji**
**Ube-shi, Yamaguchi 755-8633 (JP)**
- **Katagiri, Naoya**
**Ube-shi, Yamaguchi 755-8633 (JP)**
- **Kugimoto, Junichi**
**Ube-shi, Yamaguchi 755-8633 (JP)**

(74) Representative: **Plougmann Vingtoft a/s**
**Strandvejen 70**
**2900 Hellerup (DK)**

(56) References cited:
**EP-A1- 0 962 440** **EP-A2- 1 225 215**
**WO-A1-2011/054809** **WO-A2-2009/025939**
**DE-A1- 19 832 016** **JP-A- H09 124 594**
**US-A- 4 675 450**

- **GENGXIU ZHENG ET AL: "Metal-Free: An Efficient and Selective Catalytic Aerobic Oxidation of Hydrocarbons with Oxime and N -Hydroxyphthalimide", ADVANCED SYNTHESIS & CATALYSIS, vol. 351, no. 16, 1 November 2009 (2009-11-01), pages 2638-2642, XP055098757, ISSN: 1615-4150, DOI: 10.1002/adsc.200900509**
- **TORIBIO P P ET AL: "Ethylbenzene oxidation to its hydroperoxide in the presence of N-hydroxyimides and minute amounts of sodium hydroxide", APPLIED CATALYSIS A: GENERAL, ELSEVIER SCIENCE, AMSTERDAM, NL, vol. 363, no. 1-2, 1 July 2009 (2009-07-01), pages 32-39, XP026223784, ISSN: 0926-860X, DOI: 10.1016/J.APCATA.2009.04.023 [retrieved on 2009-04-18]**
- **GALEN J SUPPES ET AL: "Solvent and catalytic metal effects on the decomposition of cumene hydroperoxide", INDUSTRIAL & ENGINEERING CHEMISTRY RESEARCH, AMERICAN CHEMICAL SOCIETY, US, vol. 28, no. 8, 1 January 1989 (1989-01-01), pages 1146-1152, XP002593917, ISSN: 0888-5885**
- **DATABASE WPI Week 201107 Thomson Scientific, London, GB; AN 2011-A94740 XP002730740, & RU 2 409 548 C1 (AS SIBE CATALYSIS INST) 20 January 2011 (2011-01-20)**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

TECHNICAL FIELD

The present invention relates to a method for oxidizing a hydrocarbon compound with molecular oxygen to produce an alcohol and/or a ketone.

BACKGROUND ART

Oxidations of a hydrocarbon compound with molecular oxygen, in particular with air, have been studied for years, and a number of methods are disclosed. In autoxidation of a hydrocarbon compound, oxidation of cyclohexane is particularly important for industry. The resulting cyclohexanone and cyclohexanol are highly important compounds as raw materials for Nylon-6 or Nylon-6,6.

Oxidations of a hydrocarbon compound with molecular oxygen mainly proceed via the two steps. The first step is a step in which a hydrocarbon compound is oxidized to form a hydroperoxide, and the second step is a step in which the hydroperoxide is decomposed into a ketone and an alcohol. The reaction in the second step is a radical decomposition reaction of the hydroperoxide, and the selectivity for the ketone and the alcohol in the reaction is generally lower than that in the oxidation reaction of the first step. In order to obtain a ketone and an alcohol from a hydrocarbon compound with high yield, a method in which the decomposition of the hydroperoxide formed by the oxidation reaction of the hydrocarbon compound is prevented and in which the hydroperoxide is selectively decomposed in another step is effective.

For example, in the oxidation of cyclohexane, the conversion of cyclohexane generally is kept low because the products, cyclohexanone and cyclohexanol, are a product are more susceptible to the oxidation than cyclohexane and various carboxylic acids are produced as by-products. More preferably, a method in which the decomposition of the intermediate product, cyclohexyl hydroperoxide, is prevented in the oxidation step of cyclohexane, and in which the cyclohexyl hydroperoxide is selectively decomposed in the next step is adopted. For the decomposition of cyclohexyl hydroperoxide, a process in which the reaction solution obtained by the oxidation reaction of cyclohexane is contacted with an aqueous solution of alkali such as sodium hydroxide is generally adopted.

On the other hand, the binding energy between the carbon backbone and the hydrogen atom of an alicyclic hydrocarbon such as cyclohexane (a so-called secondary C-H binding energy) is a high value of 94 kcal/mol (Non-Patent Document 1), and the alicyclic hydrocarbon is less susceptible to the oxidation with molecular oxygen. While the oxidation reaction of the alicyclic hydrocarbon at a high temperature by breaking the C-H bond of the alicyclic hydrocarbon and by oxidizing the alicyclic hydrocarbon with molecular oxygen is possible, a huge high pressure equipment is required.

Accordingly, there is a common method in which a transition metal compound as a catalyst which is soluble to a hydrocarbon compound is used as a raw material. Transition metal compounds such as long-chain carboxylic acid salts of cobalt, for example, cobalt naphthenate, cobalt octylate, cobalt laurylate, cobalt palmitate, and cobalt stearate are generally used. When a transition metal compound is used, a hydroperoxide is decomposed by the action of the transition metal, and the resulting alkoxy radical and peroxy radical accelerate the oxidation reaction of hydrocarbon compound. However, the selectivity of the oxidation reaction was not sufficient because of the side reaction as described below and the like. Therefore, it is considered that the method in which a transition metal compound is used as a catalyst is incompatible with the technological idea that the decomposition of cyclohexyl hydroperoxide is prevented in the oxidation step of cyclohexane as described above, and then the cyclohexyl hydroperoxide is selectively decomposed in the next step, and the method has a problem that the yields of cyclohexanone and cyclohexanol fall.

As the method in which the decomposition of cyclohexyl hydroperoxide is prevented in the oxidation step of cyclohexane, an oxidation method without adding a transition metal compound purposely, and a method in which a cyclohexyl hydroperoxide stabilizing agent such as phosphate diester (Patent Document 1) is added are known. On the other hand, as the method in which the oxidation reaction is accelerated, a method in which an N-hydroxy dicarboxylic acid imide such as N-hydroxy phthalimide is added (Patent Document 2 and Non-Patent Document 2), and a method in which a nitroso radical is oxidized with copper (II) to form a nitrosonium salt, and an alcohol is oxidized with the nitrosonium salt to form an aldehyde, and the nitroso radical is recycled (Non-Patent Document 3) are known.

Furthermore, stainless-steel generally used for a reactor is composed of a transition metal, such as iron, nickel, chromium. Therefore, the wall of the reactor is estimated to accelerate the decomposition of hydroperoxides. Accordingly, there is adopted a method in which the surface of the reacting unit is inactivated with pyrophosphoric acid or salt thereof, or alternatively, a method in which a reacting unit for the oxidation reaction is coated with PFA (tetrafluoroethylene-perfluoroalkyl vinyl ether copolymer) to prevent the decomposition of cyclohexyl hydroperoxide on the surface of the reacting unit (Patent Document 3).

Patent Document 5 discloses the oxidation of cyclohexane as a hydrocarbon substrate in which the oxidation product comprises cyclohexyl hydroperoxide, cyclohexanol and cyclohexanone. Cyclohexyl hydroperoxide is decomposed to cyclohexanol and cyclohexanone either thermally or with the assistance of a catalyst. The catalyst for the decomposition (cleavage) step can be a homogeneous catalyst such as an acid, a halide or an oxide, or a heterogeneous catalyst such as smectite clay. Patent Document 5 does not disclose that a serial multistage reacting unit is used in the oxidation step and that the decomposition reaction of the hydroperoxide is carried out with an aqueous alkaline solution. Moreover, Patent Document 5 does not disclose that a reacting unit for carrying out at least the final stage reaction in the oxidation step and a transferring unit to a unit for the decomposition step have an inner surface formed by a material from which no transition metal ion is generated.

PRIOR ART DOCUMENTS

PATENT DOCUMENTS

Patent Document 1: Japanese Patent Laid-Open No. 62-120359
Patent Document 2: Japanese Patent Laid-Open No. 8-38909
Patent Document 3: International Publication No. 2011-054809
Patent Document 4: Japanese Patent Laid-Open No. 2007-510724
Patent Document 5: WO2009/025939

NON-PATENT DOCUMENTS

Non-Patent Document 1: J. A. Kerr, Chem. Rev., 66, 465 (1966)
Non-Patent Document 2: J. Org. Chem. 62, 6810 (1997)
Non-Patent Document 3: J. Am. Chem. Soc. 106, 3374 (1984)

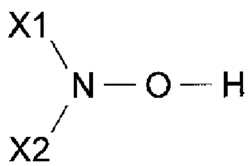

(1a)

PROBLEM TO BE SOLVED BY THE INVENTION

However, the methods as described in Patent Documents 1 and 3 have a disadvantage that oxidation rate is slow because an accelerating effect on oxidation by an alkoxy radical and a peroxy radical generated in decomposition of the hydroperoxide can be not expected. The decrease of oxidation rate results in the decrease of the productivity per a volume of an oxidation reacting unit, as well as the increase of the oxygen concentration in the waste gas, and the increase of flash ignition and explosion risks. Therefore, there is required the use of a huge reacting unit having a large depth, or the reaction at high temperature. In particular, when the oxidation temperature is set to be a high temperature, sequential oxidation of an alcohol and ketone is accelerated, and then the yield of an alcohol and/or a ketone falls. Additionally, since the vapor pressure of the hydrocarbon compound as raw material increases, there is also a problem that high pressure resisting reactor is required. In order to avoid the problem, a method in which a new reacting zone for absorbing and reacting unreacted oxygen is mounted, is contrived (Patent Document 4). However, by any method as described above, the decrease of the selectivity for ketone and alcohol, or an approach which results in the increase of the cost of the oxidation apparatus were unavoidable.

N-hydroxy phthalimide used in the method as described in Patent Document 2 and Non-Patent Document 2 is practically insoluble in alicyclic hydrocarbons, such as cyclohexane. Therefore, a polar solvent such as benzonitrile must be used, which is unfavorable for industrial application. In the method as described in Non-Patent Document 3, a large number of copper must be recycled, and Non-Patent Document 3 doesn’t describe an application for autoxidation of alicyclic hydrocarbons.

An object of the present invention is to provide a method for producing an oxide of a hydrocarbon compound safely and economically by preventing the decomposition of a hydroperoxide in the oxidation of the hydrocarbon compound with molecular oxygen to improve the yield of the oxide of the hydrocarbon compound, and by solving the problem of decreasing the oxidation rate associated with preventing the decomposition of the hydroperoxide.

Therefore, an object of the first embodiment of the present invention is to provide a method for oxidizing a hydrocarbon compound with molecular oxygen to produce an alcohol and/or a ketone. In addition, an object of the second embodiment of the present disclosure is to provide an oxidation catalyst which accelerates the oxidation of the hydrocarbon compound with molecular oxygen, and which improves the yield of at least one of an alcohol, a ketone, and a hydroperoxide having the same carbon number as the hydrocarbon compound. In addition, an object of the third embodiment of the present invention is to prevent the decomposition of the hydroperoxide in the step for oxidizing the hydrocarbon compound with molecular oxygen and to provide a method for producing a ketone and/or an alcohol with high yield.

MEANS FOR SOLVING THE PROBLEM

The present invention relates to:
A method for oxidizing a linear or cyclic saturated hydrocarbon compound with molecular oxygen to produce a ketone and/or an alcohol, comprising:

(a) oxidizing the a linear or cyclic saturated hydrocarbon compound at a temperature of 120°C or more and 160°C or less using a serial multistage reacting unit to obtain the corresponding hydroperoxide;
(b) decomposing the hydroperoxide by adding an aqueous alkaline solution to obtain the corresponding ketone and alcohol; and
(c) transferring the reaction solution obtained by the step (a) to a unit in which the step (b) is carried out;

wherein a reacting unit for carrying out at least the final stage reaction in the step (a) and a transferring unit for the step (c) have an inner surface formed by a material from which no transition metal ion is generated.

EFFECT OF THE INVENTION

According to the first embodiment of the invention, a hydrocarbon compound can be oxidized with molecular oxygen to produce at least one of an alcohol, a ketone, and a hydroperoxide having the same carbon number as the hydrocarbon compound with high yield and high productivity. Using an oxidation catalyst according to the second embodiment of the invention, at least one of an alcohol, a ketone, and a hydroperoxide having the same carbon number as the hydrocarbon compound can be produced with high yield. According to the third embodiment of the invention, the decomposing of the hydroperoxide in the step for oxidizing the hydrocarbon compound with molecular oxygen can be prevented and a method for producing a ketone and/or an alcohol with high yield can be provided.

BRIEF DESCRIPTION OF DRAWINGS

FIG. 1 is a schematic view showing a configuration of a reacting unit used in Examples 3-14 and 3-15 and Comparative Example 3-7.
FIG. 2 is a schematic view showing a configuration of a transferring unit used in Comparative Example 3-8 and Example 3-16.

MODE FOR CARRYING OUT THE INVENTION

Hereinafter, the present invention will be described in detail.

The structures of hydrocarbon compounds used in the present invention are not particularly limited. Examples of hydrocarbon compounds used in the present invention include linear saturated hydrocarbons, linear unsaturated hydrocarbons, cyclic saturated hydrocarbons, and cyclic unsaturated hydrocarbons.

The linear saturated hydrocarbon is preferably a linear saturated hydrocarbon having a carbon atom number of 3 to 10. Specific examples of the linear saturated hydrocarbon having a carbon atom number of 3 to 10 include propane, butane, pentane, hexane, heptane, octane, nonane, and decane.

The linear unsaturated hydrocarbon is preferably a linear unsaturated hydrocarbon having a carbon atom number of 3 to 6. Specific examples of the linear unsaturated hydrocarbon having a carbon atom number of 3 to 6 include propylene, butylene, pentene, and hexene.

The cyclic saturated hydrocarbon is preferably a cyclic saturated hydrocarbon having a carbon atom number of 5 to 12. Specific examples of the cyclic saturated hydrocarbon having a carbon atom number of 5 to 12 include cyclopentane, cyclohexane, cycloheptane, cyclooctane, cyclodecane, cycloundecane, and cyclododecane.

The cyclic unsaturated hydrocarbon is preferably a cyclic unsaturated hydrocarbon having a carbon atom number of 5 to 12. Specific examples of the cyclic unsaturated hydrocarbon having a carbon atom number of 5 to 12 include aliphatic cyclic unsaturated hydrocarbons which may or may not have a side chain, such as cyclopentene, cyclohexene, cyclooctene, cycloundecene, cyclododecene, cyclohexadiene, cyclooctadiene, cyclododecadiene, and cyclododecatriene; and aromatic hydrocarbons having a side chain, such as toluene, xylene, isopropyl benzene (cumene), 1-methyl-4-isopropylbenzene (p-cymene), and cyclohexylbenzene.

Examples of these hydrocarbon compounds include their constitutional isomers. These hydrocarbon compounds may have a substituent group. Examples of the substituent group include alkyl groups, aryl groups, carbonyl group, carboxyl group, amino group, nitro group, cyano group, alkoxy groups, and halogen atoms. Preferably, the total carbon atom number of the substituted hydrocarbon compound is within the range as described above.

Of these hydrocarbon compounds, examples of important hydrocarbon compounds for industry include cyclohexane, cyclododecane, toluene, xylene, isopropyl benzene (cumene), and cyclohexylbenzene.

The hydrocarbon compound used in the present invention is oxidized with molecular oxygen to be at least one of an alcohol, a ketone, and a hydroperoxide having the same carbon number as the hydrocarbon compound. The susceptibility to the oxidation of hydrocarbon compounds is primary C-H < secondary C-H < tertiary C-H. In the primary C-H, the C-H oxidation of methyl group connected to an aromatic ring is particularly useful for industry, and thereby an alcohol having the same carbon number as the hydrocarbon compound is obtained. The alcohol is eventually oxidized to a carboxylic acid. In the case of the oxidation of the secondary C-H, an alcohol, a ketone, and a hydroperoxide having the same carbon number as the hydrocarbon compound are obtained. Generally, the hydroperoxide is decomposed by alkali to generate an alcohol and a ketone. On the other hand, in the case of the oxidation of the tertiary C-H connected to an aromatic ring, a hydroperoxide having the same carbon number as the hydrocarbon compound is obtained stably. The hydroperoxide is decomposed by acid to generate phenol and a ketone.

Specifically, cyclohexanone, cyclohexanol, and cyclohexyl hydroperoxide are obtained by the oxidation of cyclohexane, and then cyclohexyl hydroperoxide is decomposed to obtain cyclohexanone and cyclohexanol. Cyclododecanone, cyclododecanol, and cyclododecyl hydroperoxide are obtained by the oxidation of cyclododecane, and then cyclododecyl hydroperoxide is decomposed to obtain cyclododecanone and cyclododecanol. Benzyl alcohol is obtained by the oxidation of toluene, and eventually oxidized to benzoic acid. 1,4-benzene dimethanol (terephthalyl alcohol) is obtained by the oxidation of xylene, and eventually oxidized to terephthalic acid. Cumyl hydroperoxide is obtained by the oxidation of isopropyl benzene, and then decomposed to generate acetone and phenol eventually. 1-phenylcyclohexyl hydroperoxide is obtained by the oxidation of cyclohexylbenzene, and then decomposed to generate cyclohexanone and phenol eventually.

<First embodiment of the present invention>

The degree of difficulty of the oxidation of hydrocarbon compounds with molecular oxygen depends on the binding energy between the skeleton carbon atom and the hydrogen atom. Generally, the C-H binding energy of hydrocarbon compounds increases in the order of tertiary carbon, secondary carbon, and primary carbon. An electron-withdrawing substituent group, an aromatic substituent group, and a carbon-carbon double bond have an effect of decreasing the C-H binding energy of a carbon connected to the substituent group or a neighboring carbon of the substituent group and the like. Therefore, linear hydrocarbons or cyclic hydrocarbons having no substituent group are generally less susceptible to the oxidation.

For example, cyclohexane has a high C-H binding energy of 94 kcal/mol (Non-Patent Document 1), and is less susceptible to the oxidation. That is the reason why cyclohexanone and cyclohexanol can't be obtained with high yield, although they are important compounds for industry.

In order to promote the oxidation of the hydrocarbon compounds which are less susceptible to the oxidation, a method in which a transition metal compound which is soluble to a hydrocarbon compound is added as an oxidation catalyst is generally performed.

The transition metal contained in the transition metal compound is not particularly limited as long as it connects with molecular oxygen to form a peroxy complex, and it is recycled by a redox reaction. Examples of the transition metal include lanthanoid elements, vanadium, chromium, molybdenum, tungsten, iron, ruthenium, cobalt, rhodium, nickel, and copper. Of these, chromium, molybdenum, iron, ruthenium, and cobalt are excellent. In particular, cobalt is suitable for industrial application.

The transition metal can be used as an oxide, an organic acid salt, an inorganic acid salt, a halide, a heteropolyacid salt, a coordination compound, or the like. In view of the solubility in a substrate, the transition metal can be suitably used as a long-chain carboxylate salt, an acetylacetonate complex, a cyclopentadienyl complex, a triaryl phosphine complex, a phthalocyanine complex, and a porphyrin complex. When cobalt (II) is selected as the transition metal, specific examples of the transition metal compound include cobalt (II) oxide, cobalt (II) acetate, cobalt (II) propionate, cobalt (II) octylate, cobalt (II) stearate, cobalt (II) nitrate, cobalt (II) sulfate, cobalt (II) chloride, cobalt (II) bromide, cobalt

(II) molybdate, cobalt (II) tungstate, cobalt (II) acetylacetonate, bis(cyclopentadienyl) cobalt (II), triphenylphosphine cobalt (II) dichloride, cobalt (II) phthalocyanine, and tetrakisphenylporphyrin cobalt (II).

However, these transition metal compounds promote a radical decomposition of an intermediate product, hydroperoxide (called as a redox decomposition). Since the selectivity for ketone and alcohol by the radical decomposition is not acceptably high enough, the target products, ketone and alcohol, were difficult to produce with high yields.

As a method to solve the problem, a method without adding any transition metal catalyst (non-catalytic oxidation method) and a method in which a cyclohexyl hydroperoxide stabilizing agent such as phosphate diester (Patent Document 1) are adopted.

As might be expected, in the above-mentioned method, the oxidation rate is drastically decreased and the productivity per a volume of an oxidation reacting unit is decreased, and safety problem, for example, the problem that oxygen concentration in the waste gas is increased to form a gas phase having a composition of its explosion region, occurs. In order to avoid these problems, the improvement of apparatus was carried out by a method in which a stirred tank flow reactor is adopted to refine air bubbles and to increase the absorption rate of oxygen, or by a method in which a column reactor is adopted and a zone for absorbing and reacting unreacted oxygen is mounted (Patent Document 4). However, the oxidation temperature must be increased eventually, and the oxidation reaction must be carried out at high temperature.

When the oxidation reaction is carried out at high temperature, the selectivity for a ketone and an alcohol by the oxidation reaction falls, and the pressure resistance of the oxidation reacting unit must be increased, resulting in the increase of the cost of the apparatus. In addition, the reaction becomes more energy intensive by the need of heating medium at higher temperature than conventional medium.

Thus, to date, the method in which the decomposition of the hydroperoxide is prevented and the rate of the oxidation reaction is secured has not been found.

The inventors investigated earnestly, and found that at least one of an alcohol, a ketone, and a hydroperoxide can be produced without accelerating the decomposition of the hydroperoxide, and with the improvement of the oxidation rate with high yield, and using by a small apparatus safely by oxidizing a hydrocarbon compound with molecular oxygen in the presence of an N-hydroxy compound represented by the general formula (1a) (not part of the invention) or the general formula (1b) (not part of the invention) and a phosphate ester represented the general formula (2) as described below.

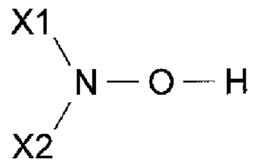 (1a)

X1=N-O-H (1b)

The N-hydroxy compound represented by the general formula (1a) or the general formula (1b) acts as an oxidation accelerating agent. In the general formula (1a) and the general formula (1b), X1 and X2 are each independently a group having a boron atom, a carbon atom, a nitrogen atom, a silicon atom, a phosphorus atom, a sulfur atom, or a halogen atom at the bond terminal; and wherein, in the general formula (1a), X1 and X2 may bind to each other to form a ring. In other words, the nitrogen atom in the N-hydroxy compound represented by the general formula (1a) and the general formula (1b) is connected with a boron atom, a carbon atom, a nitrogen atom, a silicon atom, a phosphorus atom, a sulfur atom, or a halogen atom, which is all or a part of X1 and X2.

Specific examples of the group having a carbon atom at the bond terminal include alkyl groups, alkenyl groups, alkynyl groups, cycloalkyl groups, cycloalkenyl groups, aryl groups, aralkyl groups, carbonyl group, cyano group, acyl group, formyl group, and heterocyclic groups, and X1 and X2 may bind to each other to be a divalent organic group forming a non-conjugated ring. Specific examples of the N-hydroxy compound having these X1 and X2 include dimethyl hydroxyl amine, methyl ethyl hydroxyl amine, diethyl hydroxyl amine, diphenyl hydroxyl amine, dibenzyl hydroxyl amine, N-hydroxy aziridine, and N-hydroxy azetidine.

Specific examples of the group having a nitrogen atom at the bond terminal include amino group, imino group, hydrazide group, nitro group, nitroso group, and nitrile group. Specific examples of the group having a phosphorus atom at the bond terminal include phosphino group, phosphinidene group, phosphono group, phosphinyl group, phosphonoyl group, phosphoryl group, phospho group, and phosphoro group. Specific examples of the group having a sulfur atom at the bond terminal include sulfo group, sulfonyl group, and mercapto group. Specific examples of the group having a silicon atom at the bond terminal include silyl group. Specific examples of the group having a boron atom at the bond terminal include boryl group. Specific examples of the halogen atom include fluorine atom, chlorine atom, bromine atom,

and iodine atom.

In the case of the N-hydroxy compound having a group having any atom other than carbon atom at the bond terminal as X1 or X2, the solubility of the N-hydroxy compound in hydrocarbon compounds as raw materials is decreased. Therefore, either X1 or X2 is preferably an organic group.

In the N-hydroxy compounds represented by the general formula (1a) or the general formula (1b), an oxime compound represented by the general formula (3) is preferable because the compound has a remarkable accelerating effect on the oxidation.

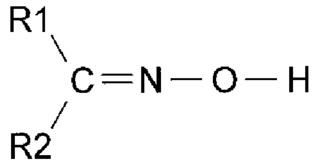

(3)

wherein, in the general formula (3), R1 and R2 are each independently a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, a substituted or unsubstituted aryl group, a substituted or unsubstituted aralkyl group, a substituted or unsubstituted heterocyclic group, or hydrogen atom; or R1 and R2 bind to each other to be a divalent organic group forming a non-conjugated ring.

The alkyl group is preferably an alkyl group having a carbon atom number of 1 to 20, is more preferably an alkyl group having a carbon atom number of 1 to 12, and is further preferably an alkyl group having a carbon atom number of 2 to 8. Specific examples of the alkyl group include methyl group, ethyl group, propyl group, isopropyl group, butyl group, isobutyl group, s-butyl group, t-butyl group, pentyl group, isopentyl group, hexyl group, isohexyl group, heptyl group, octyl group, nonyl group, decyl group, dodecyl group, and pentadecyl group.

The alkenyl group is preferably an alkenyl group having a carbon atom number of 2 to 20, is more preferably an alkenyl group having a carbon atom number of 2 to 12, and is further preferably an alkenyl group having a carbon atom number of 2 to 8. Specific examples of the alkenyl group include vinyl group, allyl group, 1-propenyl group, 1-butenyl group, 1-pentenyl group, and 1-octenyl group.

The alkynyl group is preferably an alkynyl group having a carbon atom number of 2 to 20, is more preferably an alkynyl group having a carbon atom number of 2 to 12, and is further preferably an alkynyl group having a carbon atom number of 2 to 8. Specific examples of the alkynyl group include ethynyl group and 1-propynyl group.

The cycloalkyl group is preferably a cycloalkyl group having a carbon atom number of 3 to 20, and is more preferably a cycloalkyl group having a carbon atom number of 3 to 15. Specific examples of the cycloalkyl group include cyclopropyl group, cyclobutyl group, cyclopentyl group, cyclohexyl group, cycloheptyl group, cyclooctyl group, and cyclododecyl group.

The cycloalkenyl group is preferably a cycloalkenyl group having a carbon atom number of 3 to 20, and is more preferably a cycloalkenyl group having a carbon atom number of 3 to 15. Specific examples of the cycloalkenyl group include cyclopentenyl group, cyclohexenyl group, and cyclooctenyl group.

The aryl group is preferably an aryl group having a carbon atom number of 6 to 18. Specific examples of the aryl group include phenyl group and naphthyl group.

The aralkyl group is preferably an aralkyl group having a carbon atom number of 7 to 14. Specific examples of the aralkyl group include benzyl group, 2-phenylethyl group, and 3-phenylpropyl group.

R1 and R2 may bind to each other to be a divalent organic group forming a non-conjugated ring. The ring formed is not particularly limited as long as it is a non-conjugated ring. The divalent organic group is preferably a linear or branched alkylene group, and is more preferably a linear alkylene group. The ring formed is preferably a 3- to 30-membered ring, more preferably a 4- to 20-membered ring, and further preferably a 5- to 14-membered ring.

As used herein, the non-conjugated ring means a ring other than rings forming n-electron conjugate between a C=N double bond of an oxime group and a C=C double bond in the ring. Specific examples of the oxime compound forming a conjugated ring include benzoquinone dioxime, benzoquinone monooxime (in equilibrium with nitrosophenol).

These organic groups may have a substituent group whether the organic groups form a ring or not as long as they do not inhibit the reaction. Examples of the substituent group include, but are not limited to, halogen atoms, oxo group, mercapto group, substituted oxy groups (for example, alkoxy groups, aryloxy groups, and acyoxy groups), substituted thio groups (for example, alkylthio groups, arylthio groups, and acylthio groups), substituted oxycarbonyl groups (for example, alkyloxycarbonyl groups and aryloxycarbonyl groups), substituted or unsubstituted carbamoyl groups (for example, carbamoyl group, N-alkylcarbamoyl groups, N,N-dialkylcarbamoyl groups, N-aryl oxycarbonyl groups, and N,N-diarylcarbamoyl groups), cyano group, nitro group, substituted or unsubstituted aminoalkyl groups (for example, aminoalkyl groups, N-alkylaminoalkyl groups, N,N-dialkylaminoalkyl groups, N-aryl aminoalkyl groups, and N,N-diaryl

aminoalkyl groups), alkenyl groups, alkynyl groups, cycloalkyl groups, cycloalkenyl groups, aryl groups (for example, phenyl group and naphthyl group), aralkyl groups, and heterocyclic groups.

Specific examples of the oxime compound represented by the general formula (3) in which R1 and R2 don't form a ring include acetone oxime, 2-butanone oxime (methyl ethyl ketone oxime), methyl isopropyl ketone oxime, methyl tertiary-butyl ketone oxime, di-tertiary-butyl ketone oxime, 2-pentanone oxime, 3-pentanone oxime, 1-cyclohexyl-1-propanone oxime, acetaldoxime, benzaldoxime, acetophenone oxime, benzophenone oxime, and 4-hydroxy acetophenone oxime.

Specific examples of the oxime compound represented by the general formula (3) in which R1 and R2 form a non-conjugated ring include cyclopropanone oxime, cyclobutanone oxime, cyclopentanone oxime, cyclohexanone oxime, cycloheptanone oxime, cyclooctanone oxime, cyclononanone oxime, cyclodecanone oxime, cyclododecanone oxime, cyclotridecanone oxime, cyclotetradecanone oxime, cyclopentadecanone oxime, cyclohexadecanone oxime, cyclooctadecanone oxime, and cyclononadecanone oxime.

In the oxime compounds represented by the general formula (3), a glyoxime compound having a hydroxyimino group at α-carbon represented by the general formula (4) is more preferable because the compound has a particularly remarkable accelerating effect on the oxidation.

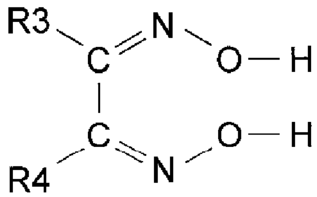

(4)

wherein, in the general formula (4), R3 and R4 are the same as R1 and R2 in the general formula (3).

Specific examples of the glyoxime compound represented by the general formula (4) include dimethyl glyoxime, methyl ethyl glyoxime, diethyl glyoxime, and diphenyl glyoxime.

In the N-hydroxy compounds represented by the general formula (1a) or the general formula (1b), an N-hydroxy dicarboxylic acid imide compound represented by the general formula (5a) or the general formula (5b) is also preferable because the compound has a particularly remarkable accelerating effect on the oxidation.

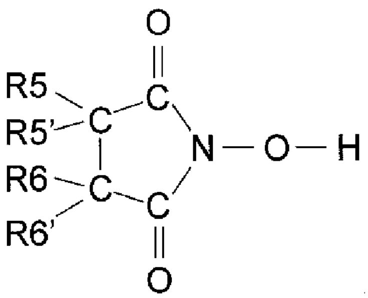

(5a)

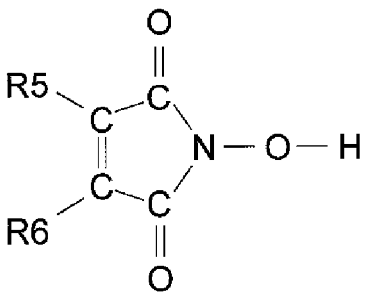

(5b)

wherein, in the general formulae (5a) and (5b), R5, R5', R6, and R6' are each independently hydrogen atom, a halogen atom, a substituted or unsubstituted alkyl group, a substituted or unsubstituted aryl group, a substituted or unsubstituted cycloalkyl group, a hydroxyl group, a substituted or unsubstituted alkoxy group, a carboxyl group, a substituted or unsubstituted alkoxycarbonyl group, or a substituted or unsubstituted acyl group; or R5 or R5' and R6 or R6' bind to each other to be a divalent organic group forming a ring.

Specific examples of the halogen atom include fluorine atom, chlorine atom, bromine atom, and iodine atom. The alkyl group, the aryl group, and the cycloalkyl group are the same as the alkyl group, the aryl group, and the cycloalkyl group of R1 or R2 in the general formula (3), respectively. The alkyl portion of the alkoxy group, the alkoxycarbonyl group, and the acyl group are the same as the alkyl group of R1 or R2 in the general formula (3).

R5 or R5' and R6 or R6' may bind to each other to be a divalent organic group forming a ring. The ring formed may be an aromatic or non-aromatic ring. The divalent organic group is preferably a linear or branched alkylene group, and is more preferably a linear alkylene group. The ring formed is preferably a 3- to 30-membered ring, more preferably a 4- to 20-membered ring, and further preferably a 5- to 14-membered ring.

These organic groups may have a substituent group whether the organic groups form a ring or not as long as they do not inhibit the reaction. The substituent group may be the same as the substituent group which R1 or R2 in the general formula (3) may have.

Specific examples of the N-hydroxy compound represented by the general formula (5a) or the general formula (5b) include N-hydroxy succinic acid imide, N-hydroxy maleic acid imide, N-hydroxyhexahydro phthalic acid imide, N,N'-dihydroxy cyclohexane tetracarboxylic acid imide, N-hydroxy phthalic acid imide, N-hydroxy tetrachloro phthalic acid imide, N-hydroxy tetrabromo phthalic acid imide, N-hydroxy het acid imide, N-hydroxy himic acid imide, N-hydroxy trimellitic acid imide, N,N'-dihydroxy pyromellitic acid imide, N,N'-dihydroxy naphthalene tetracarboxylic acid imide, and N,N'-dihydroxybiphenyl tetracarboxylic acid imide.

The N-hydroxy compound represented by the general formula (1a) or the general formula (1b) may be used alone, or may be used in combination with two or more. The added amount of the N-hydroxy compound (when used in combination with two or more, total amount of the compounds added) is preferably from 0.000001 to 0.001 mol with respect to 1 mol of the hydrocarbon compound. As the added amount of the N-hydroxy compound increases, an effect of increasing the oxidation rate becomes remarkable. However, if the amount is adjusted to 0.001 mol or less, it is economical because of saving the cost. On the other hand, if the added amount of the N-hydroxy compound is 0.000001 mol or more, the oxidation rate is estimated to increase sufficiently.

Since the N-hydroxy compound represented by the general formula (5a) or the general formula (5b) has often extremely low solubility in hydrocarbon compounds as raw materials, the compound may be added in dissolved form with a solvent. The solvent is not particularly limited as long as it dissolves the compound of the general formula (5a) or the general formula (5b). However, since the compound of the general formula (5a) or the general formula (5b) has high polarity, the solvent is preferably a polar solvent. Specific examples of the solvent include nitriles such as acetonitrile and benzonitrile; nitro compounds such as nitromethane and nitrobenzene; halogenated hydrocarbons such as 1,2-dichloroethane, chlorobenzene, and o-dichlorobenzene; phenols such as phenol and p-chlorophenol; and carboxylic acids such as formic acid, acetic acid, and chloroacetic acid.

On the other hand, the phosphate ester represented by the general formula (2) acts as a decomposition inhibitor of hydroperoxides.

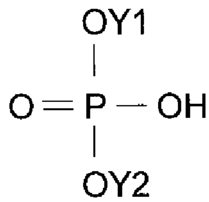

(2)

In the general formula (2), Y1 and Y2 are each independently hydrogen atom, an alkyl group having a carbon number of 4 to 12, or a cycloalkyl group having a carbon number of 5 to 12, provided that at least one of Y1 and Y2 is an alkyl group having a carbon number of 4 to 12, or a cycloalkyl group having a carbon number of 5 to 12.

Specific examples of the phosphate ester represented by the general formula (2) include monobutyl phosphate, dibutyl phosphate, monohexyl phosphate, dihexyl phosphate, mono(2-ethylhexyl) phosphate, di(2-ethylhexyl) phosphate, monooctyl phosphate, dioctyl phosphate, monododecyl phosphate, didodecyl phosphate, monocyclopentyl phosphate, dicyclopentyl phosphate, monocyclhexyl phosphate, dicyclohexyl phosphate, monocyclooctyl phosphate, dicyclooctyl phosphate, monocyclododecyl phosphate, and dicyclododecyl phosphate ester.

The phosphate ester represented by the general formula (2) may be used alone, or may be used in combination with two or more. The added amount of the phosphate ester (when used in combination with two or more, total amount of the compounds added) varies depending on the type or the amount of hydrocarbon compounds as raw materials, and when a transition metal compound is further used as an oxidation catalyst, varies depending on the type or the amount of transition metal compound, but is preferably from 0.00000001 to 0.0001 mol with respect to 1 mol of the hydrocarbon compound. If the amount of the phosphate ester is too low, the ester may not provide an effect of improving total yields of a ketone, an alcohol, and a hydroperoxide compared to the case in which phosphate ester compound is

not added. On the other hand, if the amount of the phosphate ester is too high, the improving effect is plateaued, and may be too expensive.

The mechanism that N-hydroxy compounds accelerate the oxidation reaction will be described with reference to cyclohexane as an example of hydrocarbon compound. As shown in the formula (A), the oxidation of cyclohexane is initiated by withdrawing a hydrogen of cyclohexane with an oxygen complex radical generated by a transition metal (ZOO*, Z is a transition metal atom or a complex of a transition metal atom) or with a HOO radical generated by withdrawing a hydrogen of cyclohexane with a singlet oxygen (ZOO*, Z is hydrogen atom) to generate a cyclohexyl radical. The cyclohexyl radical is then oxidized with oxygen to generate a cyclohexyl peroxy radical, and the radical withdraws a hydrogen from cyclohexane to generate a cyclohexyl radical in addition to a cyclohexyl hydroperoxide, and the radical is further oxidized.

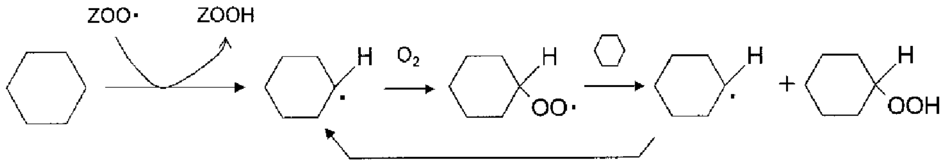

(A)

The cyclohexyl hydroperoxide generated in the formula (A) is catalytically decomposed by a transition metal. In other words, as shown in the formula (B), the cyclohexyl hydroperoxide is decomposed into a cyclohexyl alkoxy radical and a hydroxide ion by a transition metal with low oxidation state (M2+), and the transition metal is oxidized to high oxidation state (M3+). The cyclohexyl alkoxy radical withdraws a hydrogen from cyclohexane to generate a cyclohexyl radical in addition to a cyclohexanol. On the other hand, as shown in the formula (C), the transition metal with high oxidation state (M3+) decomposes cyclohexyl hydroperoxide to generate a cyclohexyl peroxy radical and a hydrogen ion, and the transition metal returns to its low oxidation state (M2+). The cyclohexyl peroxy radical withdraws a hydrogen from cyclohexane and returns to cyclohexyl hydroperoxide and generates another cyclohexyl radical. The reaction in combination of the formula (B) and the formula (C) is that, as shown in the formula (D) below, when cyclohexyl hydroperoxide is decomposed into cyclohexanol, two cyclohexyl radicals are generated from two cyclohexane molecules, and water is generated as a by-product. In other words, when cyclohexyl hydroperoxide is decomposed, new cyclohexyl radical is generated, and thereby the oxidation reaction is accelerated.

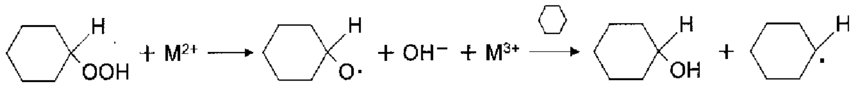
(B)

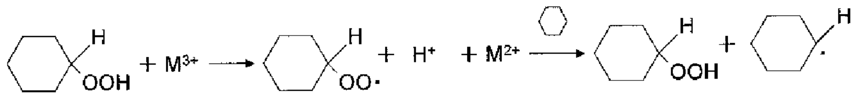
(C)

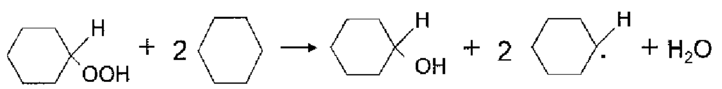
(D)

However, cyclohexyl alkoxy radical is more susceptible to the side reaction. One example is a β-ring cleavage reaction as shown in the formula (E). In the reaction, carbon-carbon bond is cleaved to generate an aldehyde, and the aldehyde is further oxidized to generate a carboxylic acid.

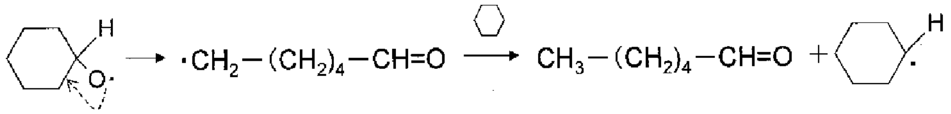
(E)

The phosphate ester reduces the side reaction such as β-ring cleavage reaction and improves the yield of oxidation by inactivating a transition metal ion and by preventing the reaction of the formulae (B) and (C). However, the generation of cyclohexyl radical is also prevented, and thereby oxidation rate is decreased. Therefore, in order to maintain the oxidation rate required for industry, the reaction temperature must be increased. However, at high temperature, as shown in the formula (F) below, cyclohexyl hydroperoxide is thermally decomposed to generate a cyclohexyl alkoxy

radical and a hydroxy radical, and radicals withdraw a hydrogen of cyclohexane to generate a cyclohexanol and a water, and to generate a cyclohexyl radical. The reaction is substantially the same as the reaction of the formula (E). Therefore, even if a transition metal ion is inactivated, when the temperature is increased to secure the reaction rate, hydroperoxide is decomposed via an alkoxy radical, and the yield is decreased (See: Yoshio Kamiya, Organic Oxidation Reaction (Yuuki Kagaku Hannou), GIHODO, published on August 5, 1973).

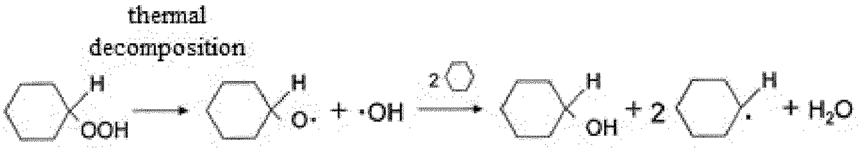


(F)

On the other hand, as shown in the formula (G), a hydrogen of an N-hydroxy compound is withdrawn by ZOO• to generate an N-oxy radical, and the N-oxy radical withdraws a hydrogen from cyclohexane to re-generate the N-hydroxy compound. At this time, a cyclohexyl radical is generated. The cyclohexyl radical is oxidized with oxygen to be a cyclohexyl peroxy radical, and the radical withdraws a hydrogen of the N-hydroxy compound to generate a cyclohexyl hydroperoxide and to generate N-oxy radical again. Therefore, in the case of an N-hydroxy compound, since a cyclohexyl radical is generated without generating a cyclohexyl alkoxy radical, the oxidation can be accelerated without falling the yield of the oxidation.

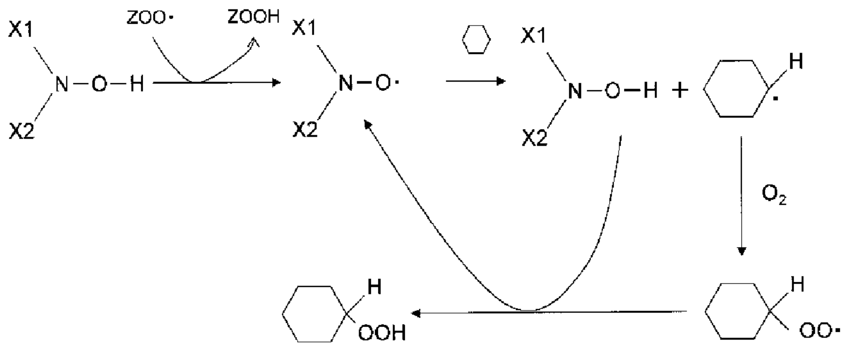

(G)

The mechanism that accelerates the oxidation reaction by N-hydroxy compounds is independent from the mechanism that accelerates the oxidation in accompanying with the decomposition of the hydroperoxide. In principle, the effect of accelerating the oxidation by N-hydroxy compounds is not affected by the used amounts of the phosphate ester and the transition metal compound. However, when the first embodiment of the present disclosure is performed, the mole ratio of the N-hydroxy compound represented by the general formula (1a) or the general formula (1b) to the phosphate ester represented by the general formula (2) is preferably 2 or more because the effect of accelerating the oxidation by the N-hydroxy compound is remarkable. On the other hand, when a transition metal compound is used as an oxidation catalyst, the mole ratio of the phosphate ester represented by the general formula (2) to the transition metal is preferably 3 or more.

For example, when the hydrocarbon compound is cyclohexane, and cobalt is used as the oxidation catalyst, the amount of the cobalt is preferably from 0.00000002 to 0.000008 mol with respect to 1 mol of cyclohexane, and the amount of the phosphate ester represented by the general formula (2) is preferably from 0.00000006 to 0.00007 mol with respect to 1 mol of cyclohexane.

As the temperature of the oxidation reaction decreases, the total yields of the ketone, the alcohol, and the hydroperoxide is increased. However, since the phosphate ester represented by the general formula (2) prevents the decomposition of the hydroperoxide, the reaction rate is decreased in the oxidation reaction at low temperature, and the oxygen concentration in the waste gas is increased to result in a safety problem. In addition, the cost of the apparatus is increased, for example, a huge reacting unit is required. Therefore, for example, when the hydrocarbon compound is cyclohexane, the oxidation reaction is preferably at 120°C or more, and is more preferably at 130°C or more.

On the other hand, if the temperature of the oxidation reaction is too high, undesirable side reactions such as a sequential oxidation of the ketone and alcohol and β-ring cleavage reaction of the hydroperoxide occurs, and then the total yield of the ketone, the alcohol, and the hydroperoxide falls. However, when the hydrocarbon compound is cyclohexane, and the reaction is carried out using a transition metal catalyst and a phosphate ester represented by the general formula (2) in the absence of an N-hydroxy compound, the oxidation reaction must be carried out at more than 165°C in order to react oxygen in the waste gas within a range of the safety level using an economical reacting unit having a large depth. However, by adding an N-hydroxy compound represented by the general formula (1a) or the

general formula (1b), the oxidation rate can be increased without decreasing the total yield of the ketone, the alcohol, and the hydroperoxide, and the oxidation reaction could be carried out at less than 165°C. Therefore, in the first embodiment of the present invention, the oxidation reaction is preferably carried out at 160°C or less so that cyclohexanone, cyclohexanol, and cyclohexyl hydroperoxide can be obtained with high yield.

As for the pressure of the oxidation reaction, as the partial pressure of oxygen increases, the concentration of the dissolved oxygen in the reaction solution is increased, which is also advantageous in view of reaction the rate and the yield of the reaction. However, the increase of the pressure of oxygen reaction results in the increase of the cost of the apparatus, and the increase of flash ignition and explosion risks. Therefore, it is economical that the oxidation reaction is carried out at a pressure from 0.01 to 0.5 MPa higher than the vapor pressure of cyclohexane at a temperature which the oxidation reaction is carried out.

The time of the oxidation reaction is determined depending on the reaction condition and the conversion of the targeted hydrocarbon compound. However, the reaction condition is selected so that the time is preferably 5 hours or less, more preferably 3 hours or less, and further preferably 2 hours or less in view of economical efficiency.

Generally, since the target products, ketones and alcohols, are more susceptible to the oxidation than hydrocarbons, a method in which the conversion of cyclohexane keeps low and the remaining cyclohexane is recovered by distillation and recycled is adopted. For example, when the hydrocarbon compound is cyclohexane, the conversion of cyclohexane is preferably set to be 2 to 15 %, and is more preferably set to be 3 to 8 %.

The reacting unit is not particularly limited, and any conventional batch reactor, half-batch reactor, and continuous reactor can be used. The continuous reactor is preferable because it has high production efficiency. The shape of the reacting unit is not also particularly limited, vertical-type reactor, tower-type reactor, stirred tank flow reactor, and pillow-type reactor can be used. In these reactors, the reaction solution is preferable in plug flow by a method in which a pillow-type reactor is sectioned into a plurality of chambers by mounting a divider perpendicular to the flow of the solution, or by a method in which a plurality of porous plates was mounted in a tower-type reactor to contact gas with liquid flowing in the opposite direction sequentially.

The hydroperoxide generated in the reaction solution for the oxidation reaction is decomposed into a ketone and an alcohol in the next step, and then the ketone and the alcohol can be obtained by distilling and recovering the unreacted hydrocarbon compound.

When the hydrocarbon compound is a cyclic saturated hydrocarbon, the hydroperoxide is decomposed by a base such as sodium hydroxide. When the hydrocarbon compound is cyclohexane, cyclohexanone and cyclohexanol are obtained via cyclohexyl hydroperoxide. When the hydrocarbon compound is cyclododecane, cyclododecanone and cyclododecanol are obtained via cyclododecyl hydroperoxide.

When the hydrocarbon compound is an aromatic hydrocarbon having a side chain, the hydroperoxide is decomposed by an acid such as sulfuric acid. When the hydrocarbon compound is cumene, phenol and acetone are obtained via cumyl hydroperoxide. When the hydrocarbon compound is p-cymene, p-cresol and acetone are obtained via cymene hydroperoxide. When the hydrocarbon compound is cyclohexyl benzene, phenol and cyclohexanone are obtained via 1-phenyl cyclohexyl hydroperoxide.

Cyclohexanol is dehydrogenated to be cyclohexanone, and then purified and used for producing ε-caprolactam. Adipic acid and oxycaproic acid generated as by-products in the oxidation reaction are also used effectively as starting materials for 1,6-hexanediol.

<Second embodiment of the present disclosure>

The oxidation catalyst according to the second embodiment of the present disclosure contains an N-hydroxy compound represented by the general formula (1a) and general formula (1b) as described above, and preferably contains an oxime compound represented by the general formula (3) as described below. The oxime compound represented by the general formula (3) can be used alone, or may be used in combination with two or more. The oxidation catalyst according to the second embodiment of the present invention has a great accelerating effect on the oxidation of hydrocarbon compounds. Even if a small amount of the oxidation catalyst is used, hydrocarbon compounds can be oxidized.

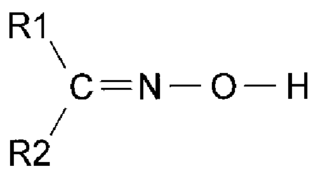

(3)

wherein, in the general formula (3), R1 and R2 are each independently a substituted or unsubstituted alkyl group, a substituted or unsubstituted alkenyl group, a substituted or unsubstituted alkynyl group, a substituted or unsubstituted

cycloalkyl group, a substituted or unsubstituted cycloalkenyl group, a substituted or unsubstituted aryl group, a substituted or unsubstituted aralkyl group, a substituted or unsubstituted heterocyclic group, or hydrogen atom; or R1 and R2 bind to each other to be a divalent organic group forming a non-conjugated ring.

The alkyl group is preferably an alkyl group having a carbon atom number of 1 to 20, is more preferably an alkyl group having a carbon atom number of 1 to 12, and is further preferably an alkyl group having a carbon atom number of 2 to 8. Specific examples of the alkyl group include methyl group, ethyl group, propyl group, isopropyl group, butyl group, isobutyl group, s-butyl group, t-butyl group, pentyl group, isopentyl group, hexyl group, isohexyl group, heptyl group, octyl group, nonyl group, decyl group, dodecyl group, and pentadecyl group.

The alkenyl group is preferably an alkenyl group having a carbon atom number of 2 to 20, is more preferably an alkenyl group having a carbon atom number of 2 to 12, and is further preferably an alkenyl group having a carbon atom number of 2 to 8. Specific examples of the alkenyl group include vinyl group, allyl group, 1-propenyl group, 1-butenyl group, 1-pentenyl group, and 1-octenyl group.

The alkynyl group is preferably an alkynyl group having a carbon atom number of 2 to 20, is more preferably an alkynyl group having a carbon atom number of 2 to 12, and is further preferably an alkynyl group having a carbon atom number of 2 to 8. Specific examples of the alkynyl group include ethynyl group and 1-propynyl group.

The cycloalkyl group is preferably a cycloalkyl group having a carbon atom number of 3 to 20, and is more preferably a cycloalkyl group having a carbon atom number of 3 to 15. Specific examples of the cycloalkyl group include cyclopropyl group, cyclobutyl group, cyclopentyl group, cyclohexyl group, cycloheptyl group, cyclooctyl group, and cyclododecyl group.

The cycloalkenyl group is preferably a cycloalkenyl group having a carbon atom number of 3 to 20, and is more preferably a cycloalkenyl group having a carbon atom number of 3 to 15. Specific examples of the cycloalkenyl group include cyclopentenyl group, cyclohexenyl group, and cyclooctenyl group.

The aryl group is preferably an aryl group having a carbon atom number of 6 to 18. Specific examples of the aryl group include phenyl group and naphthyl group.

The aralkyl group is preferably an aralkyl group having a carbon atom number of 7 to 14. Specific examples of the aralkyl group include benzyl group, 2-phenylethyl group, and 3-phenylpropyl group.

The heterocyclic group is preferably a heterocyclic group having a carbon atom number of 3 to 13. The heterocyclic group may be aromatic or non-aromatic. Specific examples of the heterocyclic group include 2-pyridyl group, 2-quinolyl group, 2-furyl group, 2-thienyl group, and 4-piperidinyl group.

R1 and R2 may bind to each other to be a divalent organic group forming a non-conjugated ring. The ring formed is not particularly limited as long as it is a non-conjugated ring. The divalent organic group is preferably a linear or branched alkylene group, and is more preferably a linear alkylene group. The ring formed is preferably a 3- to 30-membered ring, more preferably a 4- to 20-membered ring, and more further preferably a 5- to 14-membered ring.

As used herein, the non-conjugated ring means a ring other than rings forming n-electron conjugate between a C=N double bond of an oxime group and a C=C double bond in the ring. Specific examples of the oxime compound forming a conjugated ring include benzoquinone dioxime, benzoquinone monooxime (in equilibrium with nitrosophenol).

These organic groups may have a substituent group whether the organic groups form a ring or not as long as they do not inhibit the reaction. Examples of the substituent group include, but are not limited to, halogen atoms, oxo group, mercapto group, substituted oxy groups (for example, alkoxy groups, aryloxy groups, and acyoxy groups), substituted thio groups (for example, alkylthio groups, arylthio groups, and acylthio groups), substituted oxycarbonyl groups (for example, alkyloxycarbonyl groups and aryloxycarbonyl groups), substituted or unsubstituted carbamoyl groups (for example, carbamoyl group, N-alkylcarbamoyl groups, N,N-dialkylcarbamoyl groups, N-aryl oxycarbonyl groups, and N,N-diarylcarbamoyl groups), cyano group, nitro group, substituted or unsubstituted aminoalkyl groups (for example, aminoalkyl groups, N-alkylaminoalkyl groups, N,N-dialkylaminoalkyl groups, N-aryl aminoalkyl groups, and N,N-diaryl aminoalkyl groups), alkenyl groups, alkynyl groups, cycloalkyl groups, cycloalkenyl groups, aryl groups (for example, phenyl group and naphthyl group), aralkyl groups, and heterocyclic groups.

Specific examples of the oxime compound represented by the general formula (3) in which R1 and R2 don't form a ring include acetone oxime, 2-butanone oxime (methyl ethyl ketone oxime), methylisopropyl ketone oxime, methyl tertiary-butyl ketone oxime, di-tertiary-butyl ketone oxime, 2-pentanone oxime, 3-pentanone oxime, 1-cyclohexyl-1-propanone oxime, acetaldoxime, benzaldoxime, acetophenone oxime, benzophenone oxime, and 4-hydroxy acetophenone oxime.

Specific examples of the oxime compound represented by the general formula (3) in which R1 and R2 form a non-conjugated ring include cyclopropanone oxime, cyclobutanone oxime, cyclopentanone oxime, cyclohexanone oxime, cycloheptanone oxime, cyclooctanone oxime, cyclononanone oxime, cyclodecanone oxime, cyclododecanone oxime, cyclotridecanone oxime, cyclotetradecanone oxime, cyclopentadecanone oxime, cyclohexadecanone oxime, cyclooctadecanone oxime, and cyclononadecanone oxime.

In the oxime compounds represented by the general formula (3), a glyoxime compound having a hydroxyimino group at $\alpha$-carbon represented by the general formula (4) is more preferable since the compound has particularly

remarkable accelerating effect on the oxidation.

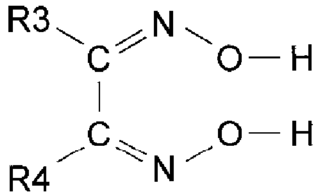 (4)

wherein, in the general formula (4), R3 and R4 are the same as R1 and R2 in the general formula (3).

Specific examples of the glyoxime compound represented by the general formula (4) include dimethyl glyoxime, methyl ethyl glyoxime, diethyl glyoxime, and diphenyl glyoxime.

The amount of the oxime compound represented by the general formula (3) is preferably from 0.000001 to 0.001 mol with respect to 1 mol of the hydrocarbon compound, and is more preferably from 0.00001 to 0.0001 mol. If the amount of the oxime compound represented by the general formula (3) is too low, an effect of accelerating the oxidation reaction is hard to obtain. In addition, if the amount of the oxime compound represented by the general formula (3) is too high, further effect of accelerating the oxidation reaction is hard to obtain, and the cost tends to increase.

In the oxidation reaction of a hydrocarbon compound by using the oxidation catalyst according to the second embodiment of the present disclosure, the following catalysis cycle seems to be accomplished. Firstly, an activated singlet oxygen withdraws a hydrogen of an oxime group in the oxime compounds represented by the general formula (3) to generate an N-oxy radical. Next, the N-oxy radical withdraws a hydrogen from a hydrocarbon compound as a substrate to generate an alkyl radical, and the N-oxy radical returns to the oxime compound. The alkyl radical is oxidized by molecular oxygen (triplet oxygen) to be a peroxy radical. The peroxy radical withdraws a hydrogen from an oxime group in the oxime compound represented by the general formula (3) to generate a hydroperoxide, and to re-generate an N-oxy radical.

Therefore, desired characteristics of an oxime compound used as an oxidation catalyst of a hydrocarbon compound is that an N-oxy radical is generated easily, and the generated N-oxy radical has high activity. The oxime compound represented by the general formula (3) meets the characteristics. On the other hand, the oxime compound in which R1 and R2 in the general formula (3) form a conjugated ring is not preferable because the generated N-oxy radical is stabilized by its resonance, and terminates the reaction conversely.

The change of the structure of N-oxy radical by intramolecular hydrogen withdrawing results in the decrease of the turnover of the catalyst. Therefore, a ketoxime compound is more suitable than an aldoxime compound as an oxime compound used for the second embodiment of the present invention because the ketoxime compound is less susceptible to intramolecular hydrogen withdrawing, and the aldoxime compound is more susceptible to intramolecular hydrogen withdrawing. Benzophenone oxime or the like is a particularly preferable oxime compound because benzophenone oxime is extremely less susceptible to intramolecular hydrogen withdrawing.

The oxidation catalyst according to the second embodiment of the present disclosure may contain an additional component as a co-catalyst other than an oxime compound represented by the general formula (3). For example, the oxidation catalyst according to the second embodiment of the present disclosure may contains a combination of an oxime compound represented by the general formula (3) and a transition metal compound. The transition metal compound can be used alone, or may be used in combination with two or more. According to the second embodiment of the present disclosure the oxidation reaction is initiated by withdrawing a hydrogen of an oxime group by a singlet oxygen. However, an energy barrier from a triplet oxygen to a singlet oxygen is high. Therefore, the yield can be improved by adding the transition metal compound to generate an oxygen complex radical, and by facilitating to initiate the reaction.

The transition metal contained in the transition metal compound is not particularly limited as long as it connects with molecular oxygen to form a peroxy complex, and it is recycled by a redox reaction. Examples of the transition metal include lanthanoid elements, vanadium, chromium, molybdenum, tungsten, iron, ruthenium, cobalt, rhodium, nickel, and copper. Of these, chromium, molybdenum, iron, ruthenium, and cobalt are excellent. In particular, cobalt is suitable for industrial application. The transition metal compound may contain one transition metal, or may contain two or more transition metals.

The transition metal compound can be used as an oxide, an organic acid salt, an inorganic acid salt, a halide, a heteropolyacid, a coordination compound of these transition metals, or the like. For example, when cobalt (II) is selected as the transition metal, specific examples of the transition metal compound include cobalt oxide, cobalt acetate, cobalt propionate, cobalt octylate, cobalt stearate, cobalt nitrate, cobalt sulfate, cobalt chloride, cobalt bromide, cobalt molybdate, cobalt tungstate, cobalt acetylacetonate, bis(cyclopentadienyl)cobalt, triphenylphosphine cobalt dichloride, cobalt phthalocyanine, and tetrakisphenylporphyrin cobalt. In view of the solubility in a substrate, long-chain carboxylate salts, acetylacetonate complexes, cyclopentadienyl complexes, triaryl phosphine complexes, phthalocyanine complexes, porphyrin complexes, and the like can be suitably used.

A transition metal compound is used as a co-catalyst, the amount thereof is preferably so as to be from 0.00000005 to 0.000010 mol of the transition metal with respect to 1 mol of the hydrocarbon compound, and is more preferably so as to be from 0.0000001 to 0.000005 mol of the transition metal. If the amount of the transition metal compound is too low, an effect of accelerating the oxidation reaction is hard to obtain. In addition, if the amount of the transition metal compound is too high, further effect of accelerating the oxidation reaction is hard to obtain, and the cost tends to increase.

Methods for oxidizing a hydrocarbon compounds may vary depending on the structure of the hydrocarbon compound as a substrate. However, the hydrocarbon compound is basically oxidized with molecular oxygen in the presence of the oxidation catalyst according to the second embodiment of the present invention. Hereinafter, a method for oxidizing cyclohexane, which is important for industry, but which is difficult to the oxidation and is hard to improve the yield of the oxidation, will be described as an example.

In the oxidation of cyclohexane, since the final products, cyclohexanone and cyclohexanol, are more susceptible to the oxidation than cyclohexane, a method in which the oxidation is terminated with a low conversion of cyclohexane, and the remaining cyclohexane is recovered by distillation and recycled to a reactor for the oxidation reaction is adopted. The conversion of cyclohexane is generally set to be 10 % or less, and many practical plants are operated so that the conversion is set to be 5 % or less.

Additionally, cyclohexyl hydroperoxide, which is a precursor of cyclohexanone and cyclohexanol, is more susceptible to thermal decomposition, and then the carbon- carbon bond is cleavaged by the thermal decomposition to generate a linear aldehyde (β-ring cleavage decomposition), and the yields of cyclohexanone and cyclohexanol eventually fall. In order to prevent the thermal decomposition of cyclohexyl hydroperoxide, cyclohexane must be oxidized at low temperature. However, when cyclohexane is oxidized at low temperature, the oxidation rate is decreased, and then a huge reactor is required to obtain cyclohexanone and cyclohexanol at the desired yields. Additionally, the decrease of the oxidation rate is not preferable in view of safety because it results in the increase of the oxygen concentration in the waste gas, and the detonating gas is also generated to increase the risk of explosion.

On the contrary, the oxidation catalyst according to the second embodiment of the present disclosure allows to oxidize cyclohexane at low temperature, and improves the yields of cyclohexane and cyclohexanol. Therefore, the oxidation reaction can be carried out in small apparatus, and results in the improvement of safety.

The temperature of the oxidation reaction is from 120°C to 160°C preferably from 130°C to 160°C. If the temperature of the oxidation reaction is too low, the reaction rate becomes slow, and thereby long-term reaction may be required to obtain a targeted product with desired yield, or huge reactor may be required. In addition, if the temperature of the oxidation reaction is too high, the yield of the targeted product tends to be decreased because of increase of by-products accompanied with carbon-carbon bond cleavage, increase of by-products by condensation reaction or coupling reaction, and increase of the sequential oxidation by-products.

The oxidation reaction is typically carried out in a pressure apparatus. For example, a batch reacting unit, a gas flow reacting unit, and a liquid-gas flow reacting unit are used. When the batch reacting unit is used, the reaction pressure is not particularly limited. However, when the gas flow reacting unit, or the liquid-gas flow reacting unit is used, air is used as an oxygen-containing gas, and cyclohexane is used as a hydrocarbon compound, the reaction is typically carried out at a pressure from 0.01 to 1 MPa higher than the vapor pressure of cyclohexane, and more practically from 0.1 to 0.5 MPa higher than the vapor pressure of cyclohexane. If the pressure is too low, the reaction rate tends to be decreased because the partial pressure of oxygen is decreased. In addition, if the pressure is too high, the cost of the apparatus becomes expensive because the wall of the reactor is required to be thick. For industrial application, the liquid-gas flow reacting unit is suitable. The oxygen-containing gas may be selected from pure oxygen, air, mixed gas by diluting pure oxygen with inert gas, if needed. Typically, air is used because it is the least expensive.

The reacting unit can be selected from any type having conventionally common shape, if needed. Specific examples of the reacting unit include pillow-shaped reactor, barrel-shaped reactor, tower-shaped reactor, and stirred reactor.

By the oxidation reaction, at least one of an alcohol, a ketone, and a hydroperoxide having the same carbon number as the hydrocarbon compound as a substrate can be produced. For example, when cyclohexane is used as a substrate, cyclohexanol, cyclohexanone, and cyclohexyl hydroperoxide are obtained.

After the oxidation reaction, a ketone and alcohol (so called KA oil) can be obtained by decomposing the hydroperoxide according to an established method, and distilling and recovering unreacted raw material.

<Third embodiment of the present invention>

In the third embodiment of the present invention, a hydrocarbon compound is oxidized with molecular oxygen (generally referred as autoxidation) to generate the corresponding hydroperoxide. Further, the hydroperoxide is decomposed to produce the corresponding ketone and/or alcohol.

Here, ketones and alcohols are more susceptible to the oxidation than hydrocarbon compounds as raw materials, and then generate further oxidized products such as carboxylic acid. Therefore, an oxidation method without adding a transition metal compound which is generally used as an oxidation catalyst such as cobalt compound (referred as non-catalytic oxidation method), and a method in which a hydroperoxide stabilizing agent such as phosphate diester is added to prevent the decomposition of the hydroperoxide in the oxidation step (Patent Document 1), and the hydroperoxide is then decomposed in the next step under non-oxidation atmosphere (referred as hydroperoxide decomposition step) are generally adopted.

Then, the decomposition of hydroperoxide is estimated to be accelerated by salts of transition metal used as an oxidation catalyst, carboxylic acid generated as by-products, as well as stainless-steel which is generally a material for a reactor. Accordingly, there is disclosed a method in which the surface of the reactor is inactivated with pyrophosphoric acid salt and the like, or a method in which a reacting unit for the oxidation reaction is coated with PFA (tetrafluoroethylene-perfluoroalkyl vinyl ether copolymer) (Patent Document 3).

However, in these methods, since the oxidation rate is slow, the temperature in the oxidation step must be increased. The oxidation step at high temperature had a problem that the yield (([molar quantity of the targeted ketone generated] + [molar quantity of the targeted alcohol generated] + [molar quantity of the targeted hydroperoxide generated]) / [molar quantity of the hydrocarbon compound consumed as a raw material] □ 100 (%)) falls, and a problem that the hydroperoxide is decomposed during transferring to an apparatus for decomposing the hydroperoxide in the oxidation reaction solution and then the yield of hydroperoxide can't be kept.

On the other hand, in the method in which an oxidation catalyst is used and the oxidation reaction is carried out at low temperature, the yield wasn't increased sufficiently in spite of the decrease of the reaction rate.

The salt of transition metal which is soluble a hydrocarbon compound generally used as an oxidation catalyst is connected to a dibasic acid generated as a by-product in the oxidation reaction to decrease the solubility in the hydrocarbon compound and to be transferred in water generated as a by-product in the oxidation reaction. Therefore, as the oxidation reaction proceeds, the action of decomposing hydroperoxide by the salt of transition metal is rapidly decreased. From the fact, the decomposition of the hydroperoxide after proceeding the oxidation reaction is mainly assumed to be accelerated by the wall surface of a reactor including a transition metal, or a small amount of transition metal ions eluted from the surface of the reactor.

Therefore, by forming the inner surface of a reacting unit for at least for the final stage in the oxidation step and the inner surface of a transferring unit for transferring the reaction solution obtained by the oxidation step to the decomposing unit for decomposing the hydroperoxide with a material from which no transition metal ion is generated, it is estimated that the decomposition of the hydroperoxide and the occurrence of the side reaction in accompanying with the decomposition can be prevented and, and the yields of final targeted products, ketone and/or alcohol, can be increased.

As the reacting unit, any conventional reacting unit such as batch-wise reacting units, continuous tank flow reacting units, and continuous column reacting units can be used. The reaction solution in the reacting unit may be stirred by a stirrer, or stirred by utilizing air lift effect of injection gas without using a stirrer. Although the reacting unit may be single, when the reacting unit is continuous, the reacting unit is preferably a serial multistage reacting unit in which a plurality of tanks is connected in series. Alternatively, serial multistage reacting unit in which a single reactor is sectioned into a plurality of chambers along with the flow of the solution can be used.

When the serial multistage reacting unit is used, all tanks may have an inner surface formed by a material from which no transition metal ion is generated; however, a reacting unit for reacting at least the final stage needs to have an inner surface formed by a material from which no transition metal ion is generated, and a reacting unit (reacting units) in the downstream of the first reactor of the serial multistage reacting unit preferably has (have) an inner surface formed by the material from which no transition metal ion is generated.

The inner surface of the reacting unit includes all portion contacted with a solution, such as the wall of the unit, a gas spray nozzle, a stirrer blade, a baffle, and a divider.

The transferring unit refers to all apparatuses, devices and pipings positioned between the outlet of the reactor and the position in which an aqueous alkaline solution for decomposition a hydroperoxide or a decomposition catalyst is added (hereinafter referred as "starting position of decomposition"). In other words, the inner surface of the transferring unit includes, for example, a feed pump, a valve, a piping positioned between the sections, and when a column or tank is presented in the section, also includes a portion contacted with a solution of the column or tank. Since the transferring unit has a contacted area with a solution bigger than that of the reacting unit, and thereby the decomposition of hydroperoxide is accelerated, the inactivation of the surface of the transferring unit is particularly important.

The unit having an inner surface formed by a material from which no transition metal ion is generated may be an apparatus formed by a material from which no transition metal ion is generated, for example, an apparatus in which the inner surface made of stainless-steel is coated with a material from which no transition metal ion is generated. The material from which no transition metal ion is generated is not particularly limited as long as it does not contain a transition metal, and is thermally and chemically stable at temperature of the oxidation reaction. Examples thereof include heat

resistance materials such as polyimide resins; ceramic powders such as silica, alumina, silicon carbide, and boron nitride, or inorganic films obtained by their precursors; fluorine resins, such as polytetrafluoroethylene, tetrafluoroethylene-hexafluoropropylene copolymer, tetrafluoroethylene-perfluoroalkyl vinyl ether copolymer, polychlorotrifluoroethylene, and Teflon (registered trademark); and glass. Of these, fluorine resin or glass is preferable. Examples of the method for coating with these materials include application, baking, coating, and lining of colorant. In particular, in view of simplicity of the application, heating and chemical resistance, and mechanical durability, fluorine resin coating or glass lining is preferable.

The oxidation step may be carried out without any catalyst, or any catalyst may be used. The oxidation reaction without any catalyst has the advantage that the decomposition of the hydroperoxide is prevented, and the yield (([molar quantity of the targeted ketone generated] + [molar quantity of the targeted alcohol generated] + [molar quantity of the targeted hydroperoxide generated]) / [molar quantity of the hydrocarbon compound consumed as a raw material] □ 100 (%)) is high. However, since the oxidation rate, in particular, the rate at the start of the oxidation, is remarkably lower than the method in which a catalyst is added, the temperature of a tank for initiating the oxidation (when tank is batch-wise reactor, the temperature at the start of the reaction) must be increased. For example, when the hydrocarbon compound is cyclohexane, since the oxygen concentration in the waste gas must be decreased in view of safety problem, and in turn the oxygen concentration for the oxidation must be decreased, the temperature of a tank for initiating the oxidation must be 165°C or more, depending on the shape of the reacting unit. Also, the temperature at the outlet of the oxidation step is typically higher than the temperature of the tank for initiating the oxidation. In this case, the hydroperoxide is thermally decomposed between the outlet of the tank for the oxidation and the starting position of decomposition, which the yield of the oxidation falls.

Therefore, according to the third embodiment of the invention, the reaction temperature in the oxidation step must be at 160°C or less, and preferably 155°C or less by any cooling method such as cooling at each oxidation tank compulsorily.

On the other hand, as the reaction temperature in the oxidation step decreases, the yield in the oxidation step increases. However, the reaction rate is decreased, and the oxygen concentration in waste gas is increased to result in a safety problem. In addition, the cost of the apparatus is increased, for example, a huge producing unit is required. Therefore, in particular, when the hydrocarbon compound is cyclohexane, the reaction temperature of the oxidation step is preferably 120°C or more in view of safety and economical.

When a catalyst is used, the oxidation reaction can be initiated at a lower temperature than as in a reaction without any catalyst. The oxidation catalyst can be selected from any conventional transition metal catalysts, if needed. Examples of the transition metal contained in the transition metal compound include vanadium, chromium, manganese, iron, cobalt, nickel, copper, molybdenum, and ruthenium. For example, the transition metal is used as a long-chain carboxylate salt or complex which is soluble to a hydrocarbon compound, such as a naphthenate salt, an octylate salt, a laurylate salt, a palmitate salt, a stearate salt, a linolate salt, an acetylacetonate complex, a dicyclopentadienyl complex, a porphyrin complex, and a phthalocyaninyl complex. When the hydrocarbon compound is cyclohexane, the transition metal catalyst is generally used as a cobalt naphthenate or a cobalt octylate.

In the third embodiment of the present invention, the oxidation rate can be increased without decreasing the yield of the oxidation by adding an N-hydroxy compound represented by any one of the general formula (3), the general formula (5a), and the general formula (5b), with or without the use of a catalyst.

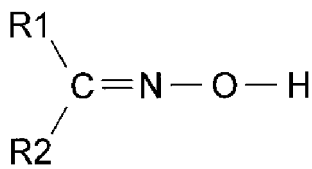

(3)

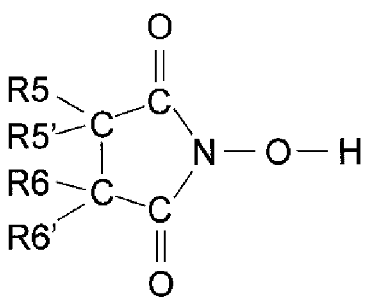

(5a)

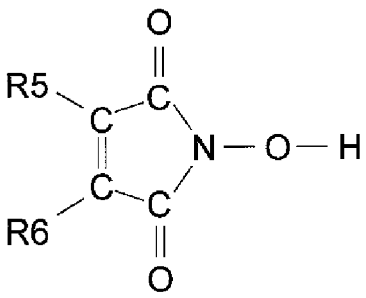

(5b)

wherein, in the general formula (3), R1 and R2 are each independently a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, a substituted or unsubstituted aryl group, a substituted or unsubstituted aralkyl group, a substituted or unsubstituted heterocyclic group, or hydrogen atom; or R1 and R2 bind to each other to be a divalent organic group forming a non-conjugated ring.

The alkyl group is preferably an alkyl group having a carbon atom number of 1 to 20, is more preferably an alkyl group having a carbon atom number of 1 to 12, and is further preferably an alkyl group having a carbon atom number of 2 to 8. Specific examples of the alkyl group include methyl group, ethyl group, propyl group, isopropyl group, butyl group, isobutyl group, s-butyl group, t-butyl group, pentyl group, isopentyl group, hexyl group, isohexyl group, heptyl group, octyl group, nonyl group, decyl group, dodecyl group, and pentadecyl group.

The alkenyl group is preferably an alkenyl group having a carbon atom number of 2 to 20, is more preferably an alkenyl group having a carbon atom number of 2 to 12, and is further preferably an alkenyl group having a carbon atom number of 2 to 8. Specific examples of the alkenyl group include vinyl group, allyl group, 1-propenyl group, 1-butenyl group, 1-pentenyl group, and 1-octenyl group.

The alkynyl group is preferably an alkynyl group having a carbon atom number of 2 to 20, is more preferably an alkynyl group having a carbon atom number of 2 to 12, and is further preferably an alkynyl group having a carbon atom number of 2 to 8. Specific examples of the alkynyl group include ethynyl group and 1-propynyl group.

The cycloalkyl group is preferably a cycloalkyl group having a carbon atom number of 3 to 20, and is more preferably a cycloalkyl group having a carbon atom number of 3 to 15. Specific examples of the cycloalkyl group include cyclopropyl group, cyclobutyl group, cyclopentyl group, cyclohexyl group, cycloheptyl group, cyclooctyl group, and cyclododecyl group.

The cycloalkenyl group is preferably a cycloalkenyl group having a carbon atom number of 3 to 20, and is more preferably a cycloalkenyl group having a carbon atom number of 3 to 15. Specific examples of the cycloalkenyl group include cyclopentenyl group, cyclohexenyl group, and cyclooctenyl group.

The aryl group is preferably an aryl group having a carbon atom number of 6 to 18. Specific examples of the aryl group include phenyl group and naphthyl group.

The aralkyl group is preferably an aralkyl group having a carbon atom number of 7 to 14. Specific examples of the aralkyl group include benzyl group, 2-phenylethyl group, and 3-phenylpropyl group.

The heterocyclic group is preferably a heterocyclic group having a carbon atom number of 3 to 13. The heterocyclic group may be aromatic or non-aromatic. Specific examples of the heterocyclic group include 2-pyridyl group, 2-quinolyl group, 2-furyl group, 2-thienyl group, and 4-piperidinyl group.

R1 and R2 may bind to each other to be a divalent organic group forming a non-conjugated ring. The ring formed is not particularly limited as long as it is a non-conjugated ring. The divalent organic group is preferably a linear or branched alkylene group, and is more preferably a linear alkylene group. The ring formed is preferably a 3- to 30-membered ring, more preferably a 4- to 20-membered ring, and further preferably a 5- to 14-membered ring.

As used herein, the non-conjugated ring means a ring other than rings forming n-electron conjugate between a C=N double bond of an oxime group and a C=C double bond in the ring. Specific examples of the oxime compound forming a conjugated ring include benzoquinone dioxime, benzoquinone monooxime (in equilibrium with nitrosophenol).

These organic groups may have a substituent group whether the organic groups form a ring or not as long as they do not inhibit the reaction. Examples of the substituent group include halogen atoms, oxo group, mercapto group, substituted oxy groups (for example, alkoxy groups, aryloxy groups, and acyoxy groups), substituted thio groups (for example, alkylthio groups, arylthio groups, and acylthio groups), substituted oxycarbonyl groups (for example, alkyloxycarbonyl groups and aryloxycarbonyl groups), substituted or unsubstituted carbamoyl group (for example, carbamoyl group, N-alkylcarbamoyl groups, N,N-dialkylcarbamoyl groups, N-aryl oxycarbonyl groups, and N,N-diarylcarbamoyl groups), cyano group, nitro group, substituted or unsubstituted aminoalkyl groups (for example, aminoalkyl groups, N-alkylaminoalkyl groups, N,N-dialkylaminoalkyl groups, N-aryl aminoalkyl groups, and N,N-diaryl aminoalkyl group), alkenyl groups, alkynyl groups, cycloalkyl groups, cycloalkenyl groups, aryl groups (for example, phenyl group and naphthyl

group), aralkyl groups, and heterocyclic groups.

Specific examples of the N-hydroxy compound represented by the general formula (3) in which R1 and R2 don't form a ring include acetone oxime, 2-butanone oxime (methyl ethyl ketone oxime), methylisopropyl ketone oxime, methyl tertiary-butyl ketone oxime, di-tertiary-butyl ketone oxime, 2-pentanone oxime, 3-pentanone oxime, 1-cyclohexyl-1-propanone oxime, acetaldoxime, benzaldoxime, acetophenone oxime, benzophenone oxime, and 4-hydroxy acetophenone oxime.

Specific examples of the N-hydroxy compound represented by the general formula (3) in which R1 and R2 form a non-conjugated ring include cyclopropanone oxime, cyclobutanone oxime, cyclopentanone oxime, cyclohexanone oxime, cycloheptanone oxime, cyclooctanone oxime, cyclononanone oxime, cyclodecanone oxime, cyclododecanone oxime, cyclotridecanone oxime, cyclotetradecanone oxime, cyclopentadecanone oxime, cyclohexadecanone oxime, cyclooctadecanone oxime, and cyclononadecanone oxime.

In the oxime compounds represented by the general formula (3), a glyoxime compound having a hydroxyimino group at $\alpha$-carbon represented by the general formula (4) is more preferable because the compound has a particularly remarkable accelerating effect on the oxidation.

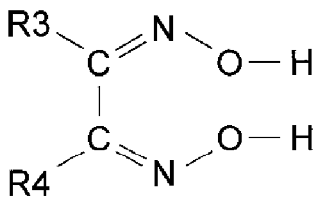

(4)

wherein, in the general formula (4), R3 and R4 are the same as R1 and R2 in the general formula (3).

Specific examples of the glyoxime compound represented by the general formula (4) include dimethyl glyoxime, methyl ethyl glyoxime, diethyl glyoxime, and diphenyl glyoxime.

In the general formulae (5a) and (5b), R5, R5', R6, and R6' are each independently hydrogen atom, a halogen atom, a substituted or unsubstituted alkyl group, a substituted or unsubstituted aryl group, a substituted or unsubstituted cycloalkyl group, a hydroxyl group, a substituted or unsubstituted alkoxy group, a carboxyl group, a substituted or unsubstituted alkoxycarbonyl group, or a substituted or unsubstituted acyl group; or R5 or R5' and R6 or R6' bind to each other to be a divalent organic group forming a ring.

Specific examples of the halogen atom include fluorine atom, chlorine atom, bromine atom, and iodine atom. The alkyl group, the aryl group, and the cycloalkyl group are the same as the alkyl group, the aryl group, and the cycloalkyl group as R1 or R2 in the general formula (3), respectively. The alkyl portion of the alkoxy group, the alkoxycarbonyl group, and the acyl group are the same as the alkyl group as R1 or R2 in the general formula (3).

R5 or R5' and R6 or R6' may bind to each other to be a divalent organic group forming a ring. The ring formed may be an aromatic or non-aromatic ring. The divalent organic group is preferably a linear or branched alkylene group, and is more preferably a linear alkylene group. The ring formed is preferably a 3- to 30-membered ring, more preferably a 4- to 20-membered ring, and further preferably a 5- to 14-membered ring.

These organic groups may have a substituent group whether the organic groups form a ring or not as long as they do not inhibit the reaction. The substituent group is the same as the substituent group which R1 or R2 in the general formula (3) may have.

Specific examples of the N-hydroxy compound represented by the general formula (5a) or the general formula (5b) include N-hydroxy succinic acid imide, N-hydroxy maleic acid imide, N-hydroxyhexahydro phthalic acid imide, N,N'-dihydroxy cyclohexane tetracarboxylic acid imide, N-hydroxy phthalic acid imide, N-hydroxy tetrachloro phthalic acid imide, N-hydroxy tetrabromo phthalic acid imide, N-hydroxy het acid imide, N-hydroxy himic acid imide, N-hydroxy trimellitic acid imide, N,N'-dihydroxy pyromellitic acid imide, N,N'-dihydroxy naphthalene tetracarboxylic acid imide, and N,N'-dihydroxybiphenyl tetracarboxylic acid imide.

The added amount of the N-hydroxy compound represented by the general formula (3), the general formula (5a), or the general formula (5b) is preferably from 0.000001 to 0.001 mol with respect to 1 mol of the hydrocarbon compound. As the added amount of the N-hydroxy compound increases, an effect of increasing the oxidation rate becomes remarkable. However, if the amount is adjusted to 0.001 mol or less, it is economical because of saving the cost. On the other hand, if the added amount of the N-hydroxy compound is 0.000001 mol or more, the oxidation rate is estimated to increase sufficiently.

Since the N-hydroxy compound represented by the general formula (5a) or the general formula (5b) has often extremely low solubility in hydrocarbon compounds as raw materials, the compound may be added in dissolved form with a solvent. The solvent is not particularly limited as long as it dissolves the compound of the general formula (5a) or the general formula (5b). However, since the compound of the general formula (5a) or the general formula (5b) has high

polarity, the solvent is preferably a polar solvent. Specific examples of the solvent include nitriles, such as acetonitrile and benzonitrile; nitro compounds, such as nitromethane and nitrobenzene; halogenated hydrocarbons, such as 1,2-dichloroethane, chlorobenzene, and o-dichlorobenzene; phenols, such as phenol and p-chlorophenol; and carboxylic acids, such as formic acid, acetic acid, and chloroacetic acid.

The hydroperoxide obtained in the oxidation step is decomposed in the next step. A ketone and/or an alcohol can be obtained by distilling and recovering the unreacted raw material, and then further distilling and purifying the material.

When the hydrocarbon compound is a cyclic saturated hydrocarbon, the hydroperoxide obtained in the oxidation step is decomposed by a base such as aqueous sodium hydroxide solution to obtain a ketone and/or an alcohol. When the hydrocarbon compound is cyclohexane, cyclohexanone and/or cyclohexanol is obtained via cyclohexyl hydroperoxide. When the hydrocarbon compound is cyclododecane, cyclododecanone and/or cyclododecanol is obtained via cyclododecyl hydroperoxide.

When the hydrocarbon compound is an aromatic hydrocarbon having a side chain, the hydroperoxide obtained in the oxidation step is decomposed by an acid such as sulfuric acid to obtain a ketone and/or an alcohol. When the hydrocarbon compound is cumene, phenol and/or acetone is obtained via cumene hydroperoxide. When the hydrocarbon compound is p-cymene, p-cresol and/or acetone are obtained via cymene hydroperoxide. When the hydrocarbon compound is cyclohexyl benzene, phenol and/or cyclohexanone is obtained via 1-phenyl cyclohexyl hydroperoxide.

Of these, cyclohexanol is dehydrogenated to be cyclohexanone, and then purified and used for producing ε-caprolactam. Adipic acid and oxycaproic acid generated as by-products in the oxidation reaction are used effectively as starting materials for 1,6-hexanediol.

EXAMPLES

Hereinafter, the present invention will be described in more detail with reference to the following Examples; however, the present invention is not limited in any way to the Examples.

<Examples according to the first embodiment of the present invention>

[Example 1-1]

To a 500ml SUS316L autoclave were added 250 g of cyclohexane, 293 mg of 0.1 % by weight solution of cobalt octylate in cyclohexane (0.2 ppm by weight as Co metal), 7 g of 1 % by weight solution of methyl ethyl ketone oxime in cyclohexane, and 2.4 g of 1.05 % by weight solution of dibutyl phosphate in cyclohexane (10 ppm by weight), and the inner temperature was increased to 157°C while introducing nitrogen from a gas injection nozzle. After the inner temperature reached at 157°C, the injected gas was changed to air. While injecting air at a speed of 1 L/min, cyclohexane was oxidized for 68 min.

After cyclohexane in the waste gas was condensed with a cooling tube and dropped into the reactor, the concentrations of carbon monoxide (CO) and carbon dioxide (CO2) in the remaining waste gas were measured, and the amounts of CO and CO2 evolved were accumulated. In addition, the resulting reaction solution was analyzed by gas chromatography (GC), and then cyclohexanone, cyclohexanol, cyclohexyl hydroperoxide, and by-products (referred as by-products detected by GC) were quantified.

Additionally, by-product carboxylic acids were extracted with a weak aqueous alkaline solution. The measurement of Total Organic Carbon (TOC) in water phase was converted to the mol number of cyclohexane by subtracting the amount of by-products detected by GC. For adipic acid and oxycaproic acid, they were separately treated with diazomethane gas to convert their methyl esters, and quantified by GC.

The conversion of cyclohexane and the yields of products were calculated by the following equations.

Conversion of cyclohexane (%) = [converted molar quantity of cyclohexane from that of all products] / [molar quantity of cyclohexane loaded] □ 100

Yield of product (%) = [converted molar quantity of cyclohexane from that of product] / [converted molar quantity of cyclohexane from that of all products] □ 100

[Converted molar quantity of cyclohexane from that of all products] = [sum of molar quantity of products detected by GC (in the case of by-products detected by GC, converted molar quantity of cyclohexane)] + [converted molar quantity of cyclohexane from TOC] + [converted molar quantity of cyclohexane from that of CO and CO2]

The result showed that the conversion of cyclohexane was 4.6 %, the yield of cyclohexanone was 10.9 %, the yield of cyclohexanol was 21.7 %, and the yield of cyclohexyl hydroperoxide was 53.9 %. Additionally, the yield of adipic acid was 1.6 %, and the yield of oxycaproic acid was 5.1 %.

[Example 1-2]

The oxidation of cyclohexane was carried out in the same manner as in Example 1-1 except that the reaction time was changed to 94 min. The result showed that the conversion of cyclohexane was 8.8 %, the yield of cyclohexanone was 16.5 %, the yield of cyclohexanol was 27.0 %, and the yield of cyclohexyl hydroperoxide was 33.8 %. Additionally, the yield of adipic acid was 2.9 %, and the yield of oxycaproic acid was 5.3 %.

[Example 1-3]

The oxidation of cyclohexane was carried out in the same manner as in Example 1-1 except that the added amount of 1 % by weight solution of methyl ethyl ketone oxime in cyclohexane was changed to 2.5 g, and that the reaction time was changed to 81 min. The result showed that the conversion of cyclohexane was 4.6 %, the yield of cyclohexanone was 11.2 %, the yield of cyclohexanol was 20.2 %, and the yield of cyclohexyl hydroperoxide was 55.2 %. Additionally, the yield of adipic acid was 1.4 %, and the yield of oxycaproic acid was 5.2 %.

[Example 1-4]

The oxidation of cyclohexane was carried out in the same manner as in Example 1-3 except that the reaction time was changed to 106 min. The result showed that the conversion of cyclohexane was 9.0 %, the yield of cyclohexanone was 17.0 %, the yield of cyclohexanol was 27.1 %, and the yield of cyclohexyl hydroperoxide was 33.2 %. Additionally, the yield of adipic acid was 2.5 %, and the yield of oxycaproic acid was 5.6 %.

[Example 1-5]

The oxidation of cyclohexane was carried out in the same manner as in Example 1-1 except that 0.33 g of 21.2 % by weight solution of N-hydroxysuccinimide in acetic acid was added instead of 7 g of 1 % by weight solution of methyl ethyl ketone oxime in cyclohexane, and that the reaction time was changed to 70 min. The result showed that the conversion of cyclohexane was 4.3 %, the yield of cyclohexanone was 10.3 %, the yield of cyclohexanol was 20.6 %, and the yield of cyclohexyl hydroperoxide was 56.2 %. Additionally, the yield of adipic acid was 1.5 %, and the yield of oxycaproic acid was 5.0 %.

[Example 1-6]

The oxidation of cyclohexane was carried out in the same manner as in Example 1-5 except that the reaction time was changed to 97 min. The result showed that the conversion of cyclohexane was 9.1 %, the yield of cyclohexanone was 17.7 %, the yield of cyclohexanol was 27.6 %, and the yield of cyclohexyl hydroperoxide was 32.0 %. Additionally, the yield of adipic acid was 2.7 %, and the yield of oxycaproic acid was 5.3 %.

[Example 1-7]

The oxidation of cyclohexane was carried out in the same manner as in Example 1-1 except that 0.07 g of N-hydroxysuccinimide as a solid was added instead of 7 g of 1 % by weight solution of methyl ethyl ketone oxime in cyclohexane, and that the reaction time was changed to 77 min. The result showed that the conversion of cyclohexane was 4.6 %, the yield of cyclohexanone was 9.5 %, the yield of cyclohexanol was 19.9 %, and the yield of cyclohexyl hydroperoxide was 56.9 %.

[Example 1-8]

The oxidation of cyclohexane was carried out in the same manner as in Example 1-7 except that the added amount of N-hydroxysuccinimide was changed to 0.025 g, and that the reaction time was changed to 90 min. The result showed that the conversion of cyclohexane was 4.5 %, the yield of cyclohexanone was 9.5 %, the yield of cyclohexanol was 19.1 %, and the yield of cyclohexyl hydroperoxide was 57.9 %.

[Example 1-9]

The oxidation of cyclohexane was carried out in the same manner as in Example 1-1 except that 7 g of 1 % by weight of dibenzyl hydroxyl amine was added instead of 7 g of 1 % by weight solution of methyl ethyl ketone oxime in cyclohexane, and that the reaction time was changed to 85 min. The result showed that the conversion of cyclohexane was 4.7 %, the yield of cyclohexanone was 11.5 %, the yield of cyclohexanol was 20.8 %, and the yield of cyclohexyl hydroperoxide was 54.0 %.

[Example 1-10]

The oxidation of cyclohexane was carried out in the same manner as in Example 1-1 except that 0.07 g of dimethyl glyoxime as a solid was added instead of 7 g of 1 % by weight solution of methyl ethyl ketone oxime in cyclohexane, and that the reaction time was changed to 56 min. The result showed that the conversion of cyclohexane was 4.4 %, the yield of cyclohexanone was 9.3 %, the yield of cyclohexanol was 19.0 %, and the yield of cyclohexyl hydroperoxide was 58.5 %.

[Example 1-11]

The oxidation of cyclohexane was carried out in the same manner as in Example 1-1 except that 0.07 g of diphenyl glyoxime as a solid was added instead of 7 g of 1 % by weight solution of methyl ethyl ketone oxime in cyclohexane, and that the reaction time was changed to 62 min. The result showed that the conversion of cyclohexane was 4.5 %, the yield of cyclohexanone was 9.6 %, the yield of cyclohexanol was 19.3 %, and the yield of cyclohexyl hydroperoxide was 57.8 %.

[Example 1-12]

The oxidation of cyclohexane was carried out in the same manner as in Example 1-5 except that 0.1 % by weight solution of cobalt octylate in cyclohexane was not added, and that the reaction time was changed to 101 min. The result showed that the conversion of cyclohexane was 4.7 %, the yield of cyclohexanone was 9.5 %, the yield of cyclohexanol was 17.4 %, and the yield of cyclohexyl hydroperoxide was 60.5 %. Additionally, the yield of adipic acid was 0.9 %, and the yield of oxycaproic acid was 4.3 %.

[Example 1-13]

The oxidation of cyclohexane was carried out in the same manner as in Example 1-12 except that the reaction time was changed to 126 min. The result showed that the conversion of cyclohexane was 8.5 %, the yield of cyclohexanone was 14.9 %, the yield of cyclohexanol was 24.7 %, and the yield of cyclohexyl hydroperoxide was 38.2 %. Additionally, the yield of adipic acid was 2.2 %, and the yield of oxycaproic acid was 6.1 %.

[Comparative Example 1-1]

The oxidation of cyclohexane was carried out in the same manner as in Example 1-1 except that 1.05 % by weight solution of dibutyl phosphate in cyclohexane and 1% by weight solution of methyl ethyl ketone oxime in cyclohexane were not added, and that the reaction time was changed to 21 min. The result showed that the conversion of cyclohexane was 3.9 %, the yield of cyclohexanone was 16.4 %, the yield of cyclohexanol was 34.2 %, and the yield of cyclohexyl hydroperoxide was 30.2 %. Additionally, the yield of adipic acid was 1.8 %, and the yield of oxycaproic acid was 4.5 %.

[Comparative Example 1-2]

The oxidation of cyclohexane was carried out in the same manner as in Comparative Example 1-1 except that the reaction time was changed to 44 min. The result showed that the conversion of cyclohexane was 7.9 %, the yield

of cyclohexanone was 24.2 %, the yield of cyclohexanol was 38.6 %, and the yield of cyclohexyl hydroperoxide was 12.8 %. Additionally, the yield of adipic acid was 2.4 %, and the yield of oxycaproic acid was 3.0 %.

[Comparative Example 1-3]

The oxidation of cyclohexane was carried out in the same manner as in Example 1-1 except that 1 % by weight solution of methyl ethyl ketone oxime in cyclohexane was not added, and that the reaction time was changed to 107 min. The result showed that the conversion of cyclohexane was 3.9 %, the yield of cyclohexanone was 10.9 %, the yield of cyclohexanol was 18.9 %, and the yield of cyclohexyl hydroperoxide was 58.8 %. Additionally, the yield of adipic acid was 0.7 %, and the yield of oxycaproic acid was 3.7 %.

[Comparative Example 1-4]

The oxidation of cyclohexane was carried out in the same manner as in Comparative Example 1-3 except that the reaction time was changed to 142 min. The result showed that the conversion of cyclohexane was 8.6 %, the yield of cyclohexanone was 15.4 %, the yield of cyclohexanol was 26.1 %, and the yield of cyclohexyl hydroperoxide was 36.0 %. Additionally, the yield of adipic acid was 2.1 %, and the yield of oxycaproic acid was 5.4 %.

[Comparative Example 1-5]

The oxidation of cyclohexane was carried out in the same manner as in Example 1-1 except that 0.1 % by weight solution of cobalt octylate in cyclohexane, 1 % by weight solution of methyl ethyl ketone oxime in cyclohexane, and 1.05 % by weight solution of dibutyl phosphate in cyclohexane were not added, and that the reaction time was changed to 107 min. The result showed that the conversion of cyclohexane was 4.7 %, the yield of cyclohexanone was 15.9 %, the yield of cyclohexanol was 24.2 %, and the yield of cyclohexyl hydroperoxide was 44.4 %. Additionally, the yield of adipic acid was 1.3 %, and the yield of oxycaproic acid was 4.5 %.

[Comparative Example 1-6]

The oxidation of cyclohexane was carried out in the same manner as in Comparative Example 1-5 except that the reaction time was changed to 138 min. The result showed that the conversion of cyclohexane was 8.7 %, the yield of cyclohexanone was 19.0 %, the yield of cyclohexanol was 27.8 %, and the yield of cyclohexyl hydroperoxide was 29.1 %. Additionally, the yield of adipic acid was 2.7 %, and the yield of oxycaproic acid was 5.3 %.

[Comparative Example 1-7]

The oxidation of cyclohexane was carried out in the same manner as in Example 1-12 except that 21.2 % by weight solution of N-methylsuccinimide in acetic acid was not added, and that the reaction time was changed to 116 min. The result showed that the conversion of cyclohexane was 2.4 %, the yield of cyclohexanone was 5.3 %, the yield of cyclohexanol was 12.4 %, and the yield of cyclohexyl hydroperoxide was 74.2 %. Additionally, the yield of adipic acid was 0.4 %, and the yield of oxycaproic acid was 3.0 %.

[Comparative Example 1-8]

The oxidation of cyclohexane was carried out in the same manner as in Comparative Example 1-7 except that the reaction time was changed to 164 min. The result showed that the conversion of cyclohexane was 8.4 %, the yield of cyclohexanone was 13.1 %, the yield of cyclohexanol was 23.9 %, and the yield of cyclohexyl hydroperoxide was 40.8 %. Additionally, the yield of adipic acid was 1.9 %, and the yield of oxycaproic acid was 5.8 %.

The reaction conditions and reaction results of Examples 1-1 to 1-13 and Comparative Examples 1-1 to 1-8 are shown in TABLE 1. In addition, the reaction time and total yield in which the conversion of cyclohexane reaches 4 % was calculated with interpolation method or extrapolation method from the results obtained in Examples or Comparative Examples in which only the conversion of cyclohexane is different, and the calculated results are also shown in TABLE 1 (Ex. 1-5 - Ex. 1-9, Ex. 1-2 and Ex. 1-13 are reference examples).

TABLE 1

| | oxidation accelerating agent | | Co catalyst (ppm) | dibutyl phosphate (ppm) | reaction time (min) | Cx conversion (%) | yield (%) | | | | at Cx conversion of 4 % | |
|---|---|---|---|---|---|---|---|---|---|---|---|---|
| | oxidation accelerating agent / solvent | I (ppm) | | | | | ON | OL | CHP | total | time (min) | yield (%) |
| Ex. 1-1 | methyl ethyl ketone oxime / Cx | 280 | 0.2 | 10 | 68 | 4.6 | 10.9 | 21.7 | 53.9 | 86.5 | 64.3 | 87.8 |
| Ex. 1-2 | methyl ethyl ketone oxime / Cx | 280 | 0.2 | 10 | 94 | 8.8 | 16.5 | 27.0 | 33.8 | 77.3 | | |
| Ex. 1-3 | methyl ethyl ketone oxime / Cx | 100 | 0.2 | 10 | 81 | 4.6 | 11.2 | 20.2 | 55.2 | 86.6 | 77.6 | 87.9 |
| Ex. 1-4 | methyl ethyl ketone oxime / Cx | 100 | 0.2 | 10 | 106 | 9.0 | 17.0 | 27.1 | 33.2 | 77.3 | | |
| Ex. 1-5 | N-methylsuccinimide / acetic acid | 280 | 0.2 | 10 | 70 | 4.3 | 10.3 | 20.6 | 56.2 | 87.1 | 68.3 | 87.7 |
| Ex. 1-6 | N-methylsuccinimide / acetic acid | 280 | 0.2 | 10 | 97 | 9.1 | 17.7 | 27.6 | 32.0 | 77.3 | | |
| Ex. 1-7 | N-methylsuccinimide / - | 280 | 0.2 | 10 | 77 | 4.6 | 9.5 | 19.9 | 56.9 | 86.3 | - | - |
| Ex. 1-8 | N-methylsuccinimide / - | 100 | 0.2 | 10 | 90 | 4.5 | 9.5 | 19.1 | 57.9 | 86.5 | - | - |
| Ex. 1-9 | dibenzyl hydroxyl amine / Cx | 280 | 0.2 | 10 | 85 | 4.7 | 11.5 | 20.8 | 54.0 | 86.3 | - | - |
| Ex. 1-10 | dimethyl glyoxime / - | 280 | 0.2 | 10 | 56 | 4.4 | 9.3 | 19.0 | 58.5 | 86.8 | - | - |
| Ex. 1-11 | diphenyl glyoxime / - | 280 | 0.2 | 10 | 62 | 4.5 | 9.6 | 19.3 | 57.8 | 86.7 | - | - |
| Ex. 1-12 | N-methylsuccinimide / acetic acid | 280 | 0 | 10 | 101 | 4.7 | 9.5 | 17.4 | 60.5 | 87.4 | 96.4 | 89.2 |
| Ex. 1-13 | N-methylsuccinimide / acetic acid | 280 | 0 | 10 | 126 | 8.5 | 14.9 | 24.7 | 38.2 | 77.8 | | |
| Comp. Ex. 1-1 | - | 0 | 0.2 | 0 | 21 | 3.9 | 16.4 | 34.2 | 30.2 | 80.8 | 21.6 | 80.7 |
| Comp. Ex. 1-2 | - | 0 | 0.2 | 0 | 44 | 7.9 | 24.2 | 38.6 | 12.8 | 75.6 | | |

(continued)

| | oxidation accelerating agent | | Co catalyst (ppm) | dibutyl phosphate (ppm) | reaction time (min) | Cx conversion (%) | yield (%) | | | | at Cx conversion of 4 % | |
|---|---|---|---|---|---|---|---|---|---|---|---|---|
| | oxidation accelerating agent / solvent | I (ppm) | | | | | ON | OL | CHP | total | time (min) | yield (%) |
| Comp. Ex. 1-3 | - | 0 | 0.2 | 10 | 107 | 3.9 | 10.9 | 18.9 | 58.8 | 88.6 | 107.7 | 88.4 |
| Comp. Ex. 1-4 | - | 0 | 0.2 | 10 | 142 | 8.6 | 15.4 | 26.1 | 36.0 | 77.5 | | |
| Comp. Ex. 1-5 | - | 0 | 0 | 0 | 107 | 4.7 | 15.9 | 24.2 | 44.4 | 84.5 | 101.6 | 86 |
| Comp. Ex. 1-6 | - | 0 | 0 | 0 | 138 | 8.7 | 19.0 | 27.8 | 29.1 | 75.9 | | |
| Comp. Ex. 1-7 | - | 0 | 0 | 10 | 116 | 2.4 | 5.3 | 12.4 | 74.2 | 91.9 | 128.8 | 88.1 |
| Comp. Ex. 1-8 | - | 0 | 0 | 10 | 164 | 8.4 | 13.1 | 23.9 | 40.8 | 77. 8 | | |

abbreviations Cx: cyclohexane, ON: cyclohexanone, OL: cyclohexanol, CHP: cyclohexyl hydroperoxide

<Examples according to the second embodiment of the present invention>

[Example 2-1]

To a 50 ml stirred autoclave were added 4.87 g of cyclohexane, 0.14 g of 0.1 % by weight solution of methyl ethyl ketone oxime in cyclohexane (ratio of methyl ethyl ketone oxime / cyclohexane: 0.000027 (mol/mol)). After that, the autoclave was sealed under air, and inner temperature was increased to 140°C. After the inner temperature reached at 140°C, the autoclave was compressed with air to a total pressure of 1.4 MPa, and a stirring was carried out for 120 min to oxidize cyclohexane. The resulting reaction solution was analyzed by gas chromatography. The result showed that the yield of cyclohexanone was 2.0 %, the yield of cyclohexanol was 2.0 %, the yield of cyclohexyl hydroperoxide was 0.4 %, and total yield of these three components was 4.4 %. It is noted that in Examples according to the second embodiment of the present invention (Examples 2-1 to Example 2-12) and Comparative Examples (Comparative Example 2-1 to Comparative Example 2-6), the yield of each product was calculated by the following equation because the amount of the reaction solution was small and the quantitative determination of all products was difficult.

Yield of product (%) = [molar quantity of product] / [molar quantity of cyclohexane introduced into reactor] □ 100

[Example 2-2]

The oxidation of cyclohexane was carried out in the same manner as in Example 2-1 except that 0.14 g of 0.1 % by weight solution of methyl ethyl ketone oxime in cyclohexane was replaced by 0.17 g of 0.1 % by weight solution of cyclohexanone oxime in cyclohexane (ratio of cyclohexanone oxime / cyclohexane: 0.000026 (mol/mol)). The result showed that the yield of cyclohexanone was 1.7 %, the yield of cyclohexanol was 1.6 %, the yield of cyclohexyl hydroperoxide was 0.4 %, and the total yield of these three components was 3.7 %.

[Example 2-3]

The oxidation of cyclohexane was carried out in the same manner as in Example 2-1 except that 0.14 g of 0.1 % by weight solution of methyl ethyl ketone oxime in cyclohexane was replaced by 0.30 g of 0.1 % by weight solution of cyclododecanone oxime in cyclohexane (ratio of cyclododecanone oxime / cyclohexane: 0.000026(mol/mol)). The result showed that the yield of cyclohexanone was 1.7 %, the yield of cyclohexanol was 1.3 %, the yield of cyclohexyl hydroperoxide was 0.3 %, and the total yield of these three components was 3.3 %.

[Example 2-4]

The oxidation of cyclohexane was carried out in the same manner as in Example 2-1 except that 0.14 g of 0.1 % by weight solution of methyl ethyl ketone oxime in cyclohexane was replaced by 0.31 g of 0.1 % by weight solution of benzophenone oxime in cyclohexane (ratio of benzophenone oxime / cyclohexane: 0.000025 (mol/mol)). The result showed that the yield of cyclohexanone was 1.8 %, the yield of cyclohexanol was 1.6 %, the yield of cyclohexyl hydroperoxide was 0.5 %, and the total yield of these three components was 3.9 %.

[Comparative Example 2-1]

The oxidation of cyclohexane was carried out in the same manner as in Example 2-1 except that 0.1 % by weight solution of methyl ethyl ketone oxime in cyclohexane was not added. The result showed that the yield of cyclohexanone was 0.8 %, the yield of cyclohexanol was 0.3 %, the yield of cyclohexyl hydroperoxide was 0.2 %, and the total yield of these three components was 1.3 %.

[Example 2-5]

The oxidation of cyclohexane was carried out in the same manner as in Example 2-4 except that the added amount of cyclohexane was changed to 4.41 g, and that 0.30 g of 0.01 % by weight solution of cobalt octylate in cyclohexane was further added (ratio of benzophenone oxime / cyclohexane: 0.000026 (mol/mol), and ratio of cobalt octylate / cyclohexane: 0.0000015 (mol/mol)). The result showed that the yield of cyclohexanone was 2.3 %, the yield of cyclohexanol was 3.1 %, the yield of cyclohexyl hydroperoxide was 0.3 %, and the total yield of these three components was 5.7 %.

[Example 2-6]

The oxidation of cyclohexane was carried out in the same manner as in Example 2-1 except that the added amount of cyclohexane was changed to 4.59 g, and that 0.30 g of 0.01 % by weight solution of cobalt octylate in cyclohexane was further added (ratio of methyl ethyl ketone oxime / cyclohexane: 0.000027 (mol/mol), and ratio of cobalt octylate / cyclohexane: 0.0000015 (mol/mol)). The result showed that the yield of cyclohexanone was 1.9 %, the yield of cyclohexanol was 2.9 %, the yield of cyclohexyl hydroperoxide was 0.1 %, and the total yield of these three components was 4.9 %.

[Example 2-7]

The oxidation of cyclohexane was carried out in the same manner as in Example 2-2 except that the added amount of cyclohexane was changed to 4.55 g, and that 0.30 g of 0.01 % by weight solution of cobalt octylate in cyclohexane was further added (ratio of cyclohexanone oxime / cyclohexane: 0.000025 (mol/mol), and ratio of cobalt octylate / cyclohexane: 0.0000015(mol/mol)). The result showed that the yield of cyclohexanone was 2.1 %, the yield of cyclohexanol was 2.2 %, the yield of cyclohexyl hydroperoxide was 0.3 %, and the total yield of these three components was 4.6 %.

[Example 2-8]

The oxidation of cyclohexane was carried out in the same manner as in Example 2-3 except that the added amount of cyclohexane was changed to 4.41 g, and that 0.30 g of 0.01 % by weight solution of cobalt octylate in cyclohexane was further added (ratio of cyclododecanone oxime / cyclohexane: 0.000026 (mol/mol), and ratio of cobalt octylate / cyclohexane: 0.0000015 (mol/mol)). The result showed that the yield of cyclohexanone was 2.1 %, the yield of cyclohexanol was 2.2 %, the yield of cyclohexyl hydroperoxide was 0.3 %, and the total yield of these three components was 4.6 %.

[Example 2-9]

The oxidation of cyclohexane was carried out in the same manner as in Example 2-8 except that the added amount of cyclohexane was changed to 4.61 g, and that 0.14 g of 0.01 % by weight solution of cyclododecanone oxime in cyclohexane was replaced by 0.11 g of 0.01 % by weight solution of acetone oxime in cyclohexane (ratio of acetone oxime / cyclohexane: 0.000025 (mol/mol), and ratio of cobalt octylate / cyclohexane: 0.0000015 (mol/mol)). The result showed that the yield of cyclohexanone was 1.8 %, the yield of cyclohexanol was 2.1 %, the yield of cyclohexyl hydroperoxide was 0.4 %, and the total yield of these three components was 4.3 %.

[Example 2-10]

The oxidation of cyclohexane was carried out in the same manner as in Example 2-8 except that the added amount of cyclohexane was changed to 4.46 g, and that 0.14 g of 0.1 % by weight solution of cyclododecanone oxime in cyclohexane was replaced by 0.22 g of 0.1 % by weight solution of acetophenone oxime in cyclohexane (ratio of acetophenone oxime / cyclohexane: 0.000028 (mol/mol), and ratio of cobalt octylate / cyclohexane: 0.0000015 (mol/mol)). The result showed that the yield of cyclohexanone was 1.9 %, the yield of cyclohexanol was 2.6 %, the yield of cyclohexyl hydroperoxide was 0.3 %, and the total yield of these three components was 4.8 %.

[Example 2-11]

The oxidation of cyclohexane was carried out in the same manner as in Example 2-8 except that the added amount of cyclohexane was changed to 4.58 g, and that 0.14 g of 0.1 % by weight solution of cyclododecanone oxime in cyclohexane was replaced by 0.13 g of 0.1 % by weight solution of butylaldehyde oxime in cyclohexane (ratio of butylaldehyde oxime / cyclohexane: 0.000025 (mol/mol), and ratio of cobalt octylate / cyclohexane: 0.0000015 (mol/mol). The result showed that the yield of cyclohexanone was 1.9 %, the yield of cyclohexanol was 2.2 %, the yield of cyclohexyl hydroperoxide was 0.3 %, and the total yield of these three components was 4.4 %.

[Example 2-12]

The oxidation of cyclohexane was carried out in the same manner as in Example 2-8 except that the added amount of cyclohexane was changed to 4.51 g, and that 0.14 g of 0.1 % by weight solution of cyclododecanone oxime

in cyclohexane was replaced by 0.20 g of 0.1 % by weight solution of benzaldoxime in cyclohexane (ratio of benzaldoxime / cyclohexane: 0.000028 (mol/mol), and ratio of cobalt octylate / cyclohexane: 0.0000015 (mol/mol)). The result showed that the yield of cyclohexanone was 1.9 %, the yield of cyclohexanol was 2.4 %, the yield of cyclohexyl hydroperoxide was 0.4 %, and the total yield of these three components was 4.7 %.

[Comparative Example 2-2]

The oxidation of cyclohexane was carried out in the same manner as in Comparative Example 2-1 except that the added amount of cyclohexane was changed to 4.7 g, and that 0.30 g of 0.01 % by weight solution of cobalt octylate in cyclohexane was further added (ratio of cobalt octylate / cyclohexane: 0.0000015 (mol/mol)). The result showed that the yield of cyclohexanone was 1.7 %, the yield of cyclohexanol was 1.8 %, the yield of cyclohexyl hydroperoxide was 0.3 %, and the total yield of these three components was 3.8 %.

These results are summarized in TABLE 2 (Ex. 2-1 - Ex. 2-4 are reference examples).

TABLE 2

| | catalyst | | yield (%) | | | |
|---|---|---|---|---|---|---|
| | oxime compound | transition metal containing catalyst | ON | OL | CHP | total |
| Ex. 2-1 | methyl ethyl ketone oxime | none | 2.0 | 2.0 | 0.4 | 4.4 |
| Ex. 2-2 | cyclohexanone oxime | none | 1.7 | 1.6 | 0.4 | 3.7 |
| Ex. 2-3 | cyclododecanone oxime | none | 1.7 | 1.3 | 0.3 | 3.3 |
| Ex. 2-4 | benzophenone oxime | none | 1.8 | 1.6 | 0.5 | 3.9 |
| Ex. 2-5 | benzophenone oxime | cobah octylate | 2.3 | 3.1 | 0.3 | 5.7 |
| Ex. 2-6 | methyl ethyl ketone oxime | cobah octylate | 1.9 | 2.9 | 0.1 | 4.9 |
| Ex. 2-7 | cyclohexanone oxime | cobah octylate | 2.1 | 2.2 | 0.3 | 4.6 |
| Ex. 2-8 | cyclododecanone oxime | cobalt octylate | 2.1 | 2.2 | 0.3 | 4.6 |
| Ex. 2-9 | acetone oxime | cobalt octylate | 1.8 | 2.1 | 0.4 | 4.3 |
| Ex. 2-10 | acetophenone oxime | cobalt octylate | 1.9 | 2.6 | 0.3 | 4.8 |
| Ex. 2-11 | butylaldehyde oxime | cobalt octylate | 1.9 | 2.2 | 0.3 | 4.4 |
| Ex. 2-12 | benzaldoxime | cobalt octylate | 1.9 | 2.4 | 0.4 | 4.7 |
| Comp. Ex. 2-1 | none | none | 0.8 | 0.3 | 0.2 | 1.3 |
| Comp. Ex. 2-2 | none | cobalt octylate | 1.7 | 1.8 | 0.3 | 3.8 |
| abbreviations ON: cyclohexanone, OL: cyclohexanol, CHP: cyclohexyl hydroperoxide | | | | | | |

[Comparative Example 2-3]

The oxidation of cyclohexane was carried out in the same manner as in Example 2-8 except that added amount of cyclohexane was changed to 4.45 g, and that 0.14 g of 0.1 % by weight solution of cyclododecanone oxime in cyclohexane was replaced by 0.21 of 0.1 % by weight solution of 1,4-bensoquinone dioxime in cyclohexane (ratio of 1,4-benzoquinone dioxime / cyclohexane: 0.000026 (mol/mol), ratio of cobalt octylate / cyclohexane: 0.0000015 (mol/mol)). However, since the absorption of oxygen was not observed, the reaction was stopped after 40 min. The resulting reaction solution was analyzed by gas chromatography, and cyclohexanone, cyclohexanol, and cyclohexyl hydroperoxide was not detected.

[Comparative Example 2-4]

The oxidation of cyclohexane was carried out in the same manner as in Example 2-8 except that the added amount of cyclohexane was changed to 4.45 g, and that 0.14 g of 0.1 % by weight solution of cyclododecanone oxime in cyclohexane was replaced by 0.20 g of 0.1 % by weight solution of para-nitrosophenol in cyclohexane (ratio of 1,4-benzoquinone dioxime / cyclohexane: 0.000028 (mol/mol), ratio of cobalt octylate / cyclohexane: 0.0000015 (mol/mol)). However, since the absorption of oxygen was not observed, the reaction was stopped after 40 min. The resulting reaction

solution was analyzed by gas chromatography, and cyclohexanone, cyclohexanol, and cyclohexyl hydroperoxide was not detected.

[Comparative Example 2-5]

The oxidation of cyclohexane was carried out in the same manner as in Example 2-8 except that the added amount of cyclohexane was changed to 4.45 g, and that 0.14 g of 0.1 % by weight solution of cyclododecanone oxime in cyclohexane was replaced by 0.27 g of 0.1 % by weight solution of 1-nitroso-2-naphthol in cyclohexane (ratio of 1,4-benzoquinone dioxime / cyclohexane: 0.000026 (mol/mol), ratio of cobalt octylate / cyclohexane: 0.0000015 (mol/mol)). However, since the absorption of oxygen was not observed, the reaction was stopped after 40 min. The resulting reaction solution was analyzed by gas chromatography, and cyclohexanone, cyclohexanol, and cyclohexyl hydroperoxide was not detected.

[Comparative Example 2-6]

The oxidation of cyclohexane was carried out in the same manner as in Example 2-8 except that the added amount of cyclohexane was changed to 4.45 g, and that 0.14 g of 0.1 % by weight solution of cyclododecanone oxime in cyclohexane was replaced by 0.27 g of 0.1 % by weight solution of 2-nitroso-1-naphthol in cyclohexane (ratio of 1,4-benzoquinone dioxime / cyclohexane: 0.000026 (mol/mol), and ratio of cobalt octylate / cyclohexane: 0.0000015 (mol/mol)). However, since the absorption of oxygen was not observed, the reaction was stopped after 40 min. The resulting reaction solution was analyzed by gas chromatography, and cyclohexanone and cyclohexyl hydroperoxide was not detected, and a small amount of cyclohexanol was only detected (yield: 0.05 %)

<Examples according to third embodiment of the present disclosure>

[Example 3-1]

To a 500 ml reactor in which the inner surface of a SUS316L autoclave was covered with a Teflon (registered trademark) coating (all inner instruments including a top cover, a stirrer wing, a gas injection nozzle, and a thermometer sheath), 250 g of cyclohexane and 293 mg of 0.1 % by weight solution of cobalt octylate in cyclohexane (0.2 ppm by weight as Co metal) were added. The inner temperature was increased to 140°C while introducing nitrogen from a gas injection nozzle. After the inner temperature reached at 140°C, injected gas was replaced with air. While injecting air at a speed of 1 L/min, cyclohexane was oxidized for 113 min.

After cyclohexane in the waste gas was condensed with a cooling tube and dropped into the reactor, the concentrations of carbon monoxide (CO) and carbon dioxide (CO2) in the remaining waste gas were measured, and the amounts of CO and CO2 evolved were accumulated. On the other hand, the resulting reaction solution was analyzed by gas chromatography (GC), and then cyclohexanone, cyclohexanol, cyclohexyl hydroperoxide, and by-products (referred as by-products detected by GC) were quantified.

Additionally, by-product carboxylic acids were extracted with a weak aqueous alkaline solution. The measurement of Total Organic Carbon (TOC) in water phase was converted to the molar quantity of cyclohexane by subtracting the amount of by-products detected by GC. For adipic acid and oxycaproic acid, they were separately treated with diazomethane gas to convert a methyl ester, and quantified by GC.

The conversion of cyclohexane and the yield of products were calculated by the following equations.

Conversion of cyclohexane (%) = [converted molar quantity of cyclohexane from that of all products] / [molar quantity of cyclohexane loaded] □ 100

Yield of product (%) = [converted molar quantity of cyclohexane from that of product] / [converted molar quantity of cyclohexane from that of all products] □ 100

[Converted molar quantity of cyclohexane from that of all products] = [sum of molar quantity of products detected by GC (in the case of by-products detected by GC, converted molar quantity of cyclohexane)] + [converted molar quantity of cyclohexane from TOC] + [converted molar quantity of cyclohexane from that of CO and CO2]

The result showed that the conversion of cyclohexane was 3.0 %, the yield of cyclohexanone was 13.5 %, the yield of cyclohexanol was 28.6 %, and the yield of cyclohexyl hydroperoxide was 45.0 %. Additionally, the yield of adipic acid was 2.5 %, and the yield of oxycaproic acid was 4.7 %.

[Example 3-2]

The oxidation of cyclohexane was carried out in the same manner as in Example 3-1 except that the reaction time was changed to 146 min. The result showed that the conversion of cyclohexane was 6.2 %, the yield of cyclohexanone was 22.3 %, the yield of cyclohexanol was 37.6 %, and the yield of cyclohexyl hydroperoxide was 20.5 %. Additionally, the yield of adipic acid was 3.7 %, and the yield of oxycaproic acid was 5.0 %.

[Example 3-3]

The oxidation of cyclohexane was carried out in the same manner as in Example 3-1 except that the reaction time was changed to 159 min. The result showed that the conversion of cyclohexane was 8.6 %, the yield of cyclohexanone was 27.5 %, the yield of cyclohexanol was 36.6 %, and the yield of cyclohexyl hydroperoxide was 12.0 %. Additionally, the yield of adipic acid was 5.1 %, and the yield of oxycaproic acid was 4.7 %.

[Comparative Example 3-1]

The oxidation of cyclohexane was carried out in the same manner as in Example 3-1 except that a SUS316L autoclave (without a surface coating) was used as a reactor, and that the reaction time was changed to 104 min. The result showed that the conversion of cyclohexane was 2.5 %, the yield of cyclohexanone was 17.6 %, the yield of cyclohexanol was 29.3 %, and the yield of cyclohexyl hydroperoxide was 39.0 %. Additionally, the yield of adipic acid was 1.4 %, and the yield of oxycaproic acid was 2.3 %.

[Comparative Example 3-2]

The oxidation of cyclohexane was carried out in the same manner as in Comparative Example 3-1 except that the reaction time was changed to 145 min. The result showed that the conversion of cyclohexane was 7.3 %, the yield of cyclohexanone was 28.2 %, the yield of cyclohexanol was 35.8 %, and the yield of cyclohexyl hydroperoxide was 11.4 %. Additionally, the yield of adipic acid was 5.0 %, and the yield of oxycaproic acid was 4.2 %.

[Example 3-4]

The oxidation of cyclohexane was carried out in the same manner as in Example 3-1 except that the reaction temperature was changed to 157°C, and that the reaction time was changed to 23 min. The result showed that the conversion of cyclohexane was 3.9 %, the yield of cyclohexanone was 14.4 %, the yield of cyclohexanol was 32.4 %, and yield of cyclohexyl hydroperoxide was 34.5 %. Additionally, the yield of adipic acid was 2.0 %, and the yield of oxycaproic acid was 4.7 %.

[Example 3-5]

The oxidation of cyclohexane was carried out in the same manner as in Example 3-4 except that the reaction time was changed to 39 min. The result showed that the conversion of cyclohexane was 8.3 %, the yield of cyclohexanone was 21.5 %, the yield of cyclohexanol was 38.6 %, and the yield of cyclohexyl hydroperoxide was 15.2 %. Additionally, the yield of adipic acid was 2.5 %, and the yield of oxycaproic acid was 3.8 %.

[Example 3-6]

The oxidation of cyclohexane was carried out in the same manner as in Example 3-4 except that a glass autoclave was used as a reactor, and that the reaction time was changed to 22 min. The result showed that the conversion of cyclohexane was 3.4 %, the yield of cyclohexanone was 14.2 %, the yield of cyclohexanol was 33.4 %, and the yield of cyclohexyl hydroperoxide was 34.5 %. Additionally, the yield of adipic acid was 2.6 %, and the yield of oxycaproic acid was 4.9 %.

[Example 3-7]

The oxidation of cyclohexane was carried out in the same manner as in Example 3-6 except that the reaction time was changed to 43 min. The result showed that the conversion of cyclohexane was 7.3 %, the yield of cyclohexanone was 21.3 %, the yield of cyclohexanol was 40.6 %, and the yield of cyclohexyl hydroperoxide was 16.3 %. Additionally, the yield of adipic acid was 2.2 %, and the yield of oxycaproic acid was 3.2 %.

[Comparative Example 3-3]

The oxidation of cyclohexane was carried out in the same manner as in Comparative Example 3-1 except that the reaction temperature was changed to 157°C, and that the reaction time was changed to 21 min. The result showed that the conversion of cyclohexane was 3.9 %, the yield of cyclohexanone was 16.4 %, the yield of cyclohexanol was 34.2 %, and the yield of cyclohexyl hydroperoxide was 30.2 %. Additionally, the yield of adipic acid was 1.8 %, and the yield of oxycaproic acid was 4.5 %.

[Comparative Example 3-4]

The oxidation of cyclohexane was carried out in the same manner as in Comparative Example 3-3 except that the reaction time was changed to 44 min. The result showed that the conversion of cyclohexane was 7.9 %, the yield of cyclohexanone was 24.2 %, the yield of cyclohexanol was 38.6 %, and the yield of cyclohexyl hydroperoxide was 12.8 %. Additionally, the yield of adipic acid was 2.4 %, and the yield of oxycaproic acid was 3.0 %.

[Comparative Example 3-5]

The oxidation of cyclohexane was carried out in the same manner as in Example 3-1 except that the reaction temperature was changed to 162°C, and that the reaction time was changed to 16 min. The result showed that the conversion of cyclohexane was 4.0 %, the yield of cyclohexanone was 18.0 %, the yield of cyclohexanol was 38.0 %, and the yield of cyclohexyl hydroperoxide was 21.5 %. Additionally, the yield of adipic acid was 1.5 %, and the yield of oxycaproic acid was 3.0 %.

[Example 3-8]

The oxidation of cyclohexane was carried out in the same manner as in Example 3-1 except that 280 ppm by weight of methyl ethyl ketone oxime was added to the reaction solution, and that the reaction time was changed to 63 min. The result showed that the conversion of cyclohexane was 2.9 %, the yield of cyclohexanone was 13.6 %, the yield of cyclohexanol was 28.7 %, and the yield of cyclohexyl hydroperoxide was 45.2 %. Additionally, the yield of adipic acid was 2.4 %, and the yield of oxycaproic acid was 4.8 %.

[Example 3-9]

The oxidation of cyclohexane was carried out in the same manner as in Example 3-8 except that the reaction time was changed to 107 min. The result showed that the conversion of cyclohexane was 8.2 %, the yield of cyclohexanone was 27.8 %, the yield of cyclohexanol was 37.0 %, and the yield of cyclohexyl hydroperoxide was 12.1 %. Additionally, the yield of adipic acid was 6.4 %, and the yield of oxycaproic acid was 4.4 %.

[Example 3-10]

The oxidation of cyclohexane was carried out in the same manner as in Example 3-8 except that the reaction temperature was changed to 150°C, and that the reaction time was changed to 22 min. The result showed that the conversion of cyclohexane was 3.3 %, the yield of cyclohexanone was 13.4 %, the yield of cyclohexanol was 28.3 %,

and the yield of cyclohexyl hydroperoxide was 44.5 %. Additionally, the yield of adipic acid was 2.6 %, and the yield of oxycaproic acid was 5.5 %.

[Example 3-11]

The oxidation of cyclohexane was carried out in the same manner as in Example 3-10 except that the reaction time was changed to 53 min. The result showed that the conversion of cyclohexane was 8.6 %, the yield of cyclohexanone was 27.3 %, the yield of cyclohexanol was 36.4 %, and the yield of cyclohexyl hydroperoxide was 11.9 %. Additionally, the yield of adipic acid was 4.7 %, and the yield of oxycaproic acid was 3.9 %.

[Example 3-12]

The oxidation of cyclohexane was carried out in the same manner as in Example 3-1 except that a solution of N-hydroxysuccinic acid imide in acetic acid was added to the reaction solution (the added amount of N-hydroxysuccinic acid imide: 280 ppm by weight, and the added amount of acetic acid: 1320 ppm by weight), and that the reaction time was changed to 48 min. The result showed that the conversion of cyclohexane was 2.7 %, the yield of cyclohexanone was 13.6 %, the yield of cyclohexanol was 28.9 %, and the yield of cyclohexyl hydroperoxide was 45.4 %. Additionally, the yield of adipic acid was 4.1 %, and the yield of oxycaproic acid was 5.9 %.

[Example 3-13]

The oxidation of cyclohexane was carried out in the same manner as in Example 3-12 except that the reaction time was changed to 88 min. The result showed that the conversion of cyclohexane was 7.0 %, the yield of cyclohexanone was 28.6 %, the yield of cyclohexanol was 38.0 %, and the yield of cyclohexyl hydroperoxide was 12.5 %. Additionally, the yield of adipic acid was 6.1 %, and the yield of oxycaproic acid was 4.4 %.

The reaction conditions and reaction results of Examples 3-1 to 3-13 and Comparative Examples 3-1 to 3-5 are shown in TABLE 3. In addition, the reaction time and total yield in which the conversion of cyclohexane reaches 4 % was calculated with interpolation method or extrapolation method from the results obtained in Examples or Comparative Examples in which only the conversion of cyclohexane is different, and the calculated results are also shown in TABLE 3 (reference examples).

TABLE 3

| | surface treatment | concentration of catalyst | additive | | added solvent | | reaction temperature (°C) | reaction time (min) | Cx conversion (%) | yield (%) | | | | at Cx conversion of 4 % | |
|---|---|---|---|---|---|---|---|---|---|---|---|---|---|---|---|
| | | Co/ppm | compound | ppm | compound | ppm | | | | ON | OL | CHP | total | time (min) | yield (%) |
| Ex. 3-1 | TC | 0.2 | | | | | 140 | 113 | 3.0 | 13.5 | 28.6 | 45.0 | 87.1 | 123 | 85.0 |
| Ex. 3-2 | TC | 0.2 | | | | | 140 | 146 | 6.2 | 22.3 | 37.6 | 20.5 | 80.4 | | |
| Ex. 3-3 | TC | 0.2 | | | | | 140 | 159 | 8.6 | 27.5 | 36.6 | 12.0 | 76.1 | | |
| Comp. Ex. 3-1 | no treatment | 0.2 | | | | | 140 | 104 | 2.5 | 17.6 | 29.3 | 39.0 | 85.9 | 117 | 82.6 |
| Comp. Ex. 3-2 | no treatment | 0.2 | | | | | 140 | 145 | 7.3 | 28.2 | 35.8 | 11.4 | 75.4 | | |
| Ex. 3-4 | TC | 0.2 | | | | | 157 | 23 | 3.9 | 14.4 | 32.4 | 34.5 | 81.3 | 23 | 81.2 |
| Ex. 3-5 | TC | 0.2 | | | | | 157 | 39 | 8.3 | 21.5 | 38.6 | 15.2 | 75.3 | | |
| Ex. 3-6 | GL | 0.2 | | | | | 157 | 22 | 3.4 | 14.2 | 33.4 | 34.5 | 82.1 | 25 | 81.5 |
| Ex. 3-7 | GL | 0.2 | | | | | 157 | 43 | 7.3 | 21.3 | 40.6 | 16.3 | 78.2 | | |
| Comp. Ex. 3-3 | no treatment | 0.2 | | | | | 157 | 21 | 3.9 | 16.4 | 34.2 | 30.2 | 80.8 | 22 | 80.7 |
| Comp. Ex. 3-4 | no treatment | 0.2 | | | | | 157 | 44 | 7.9 | 24.2 | 38.6 | 12.8 | 75.6 | | |
| Comp. Ex. 3-5 | TC | 0.2 | | | | | 162 | 16 | 4.0 | 18.0 | 38.0 | 21.5 | 77.5 | 16 | 77.5 |
| Ex. 3-8 | TC | 0.2 | MEKO | 280 | | | 140 | 63 | 2.9 | 13.6 | 28.7 | 45.2 | 87.5 | 72 | 85.3 |
| Ex. 3-9 | TC | 0.2 | MEKO | 280 | | | 140 | 107 | 8.2 | 27.8 | 37.0 | 12.1 | 76.9 | | |
| Ex. 3-10 | TC | 0.2 | MEKO | 280 | | | 150 | 22 | 3.3 | 13.4 | 28.3 | 44.5 | 86.2 | 26 | 84.8 |
| Ex. 3-11 | TC | 0.2 | MEKO | 280 | | | 150 | 53 | 8.6 | 27.3 | 36.4 | 11.9 | 75.7 | | |

(continued)

| | surface treatment | conc entration of catalyst | additive | | added solvent | | reaction temperature (°C) | reaction time (min) | Cxconversion (%) | yield (%) | | | | at Cx conversion of 4 % | |
|---|---|---|---|---|---|---|---|---|---|---|---|---|---|---|---|
| | | Co/ppm | compound | ppm | compound | ppm | | | | ON | OL | CHP | total | time (min) | yield (%) |
| Ex. 3-12 | TC | 0.2 | NHSI | 280 | acetic acid | 1320 | 140 | 48 | 2.7 | 13.6 | 28.9 | 45.4 | 87.9 | 60 | 85.2 |
| Ex. 3-13 | TC | 0.2 | NHSI | 280 | acetic acid | 1320 | 140 | 88 | 7.0 | 28.6 | 38.0 | 12.5 | 79.0 | | |

surface treatment TC: Teflon (registered trademark) coating, GL: glass lining, no treatment: untreated SUS316L surface abbreviations MEKO: methyl ethyl ketone oxime, NHSI: N-hydroxysuccinic acid imide, Cx: cyclohexane, ON: cyclohexanone. OL: cyclohexanol. CHP: cyclohexyl hydroperoxide

[Example 3-14]

As shown in FIG. 1, three column type reactors made of SUS316L having an inner surface with glass lining were connected in series (the first tower 10: volume 1066 ml, depth 785 mm; the second tower 20: volume 863 ml, depth 635 mm; the third tower 30: volume 863 ml, depth 635 mm). Cyclohexane containing 0.1 ppm of cobalt octylate in Co equivalent was transferred with a transfer pump 11 from a cyclohexane storage tank 1 and was preheated to 161.5°C by a preheater 3, and was then transferred to the first column 10 at a speed of 3000 g/h. In the first column 10, cyclohexane was oxidized by injecting air from an air tank 4 at a speed of 109 NL/h with a mass flow controller 12. At this time, cyclohexane at room temperature was further transferred with transfer pumps 21 and 31 from a cyclohexane storage tank 2 to the second column 20 at a speed of 370 g/h and to the third column 30 at a speed of 390 g/h for while air was injected with mass flow controllers 22 and 32 from an air tank 4 to the second column 20 and the third column 30 at a speed of 85 NL/h. Further, the reaction temperature was controlled by mounting condensers 13, 23 and 33 to the waste gas tube of each tank. The reaction temperature of the first column was 160°C, that of the second column was 155°C, and that of the third column was 150°C. All of the resulting reaction solution was collected in a receiver 5, and analyzed by the same method as in Example 3-1. It is noted that an equalizing pipe 6 was connected between the receiver 5 and the third column 30. The result showed that the conversion of cyclohexane was 4.8 %, the yield of cyclohexanone was 26.1 %, the yield of cyclohexanol was 36.8 %, and the yield of cyclohexyl hydroperoxide was 18.0 %.

[Example 3-15]

The oxidation of cyclohexane was carried out in the same manner as in Example 3-14 except that a SUS316L column type reactor without glass lining was used as the first column. The result showed that the conversion of cyclohexane was 4.9 %, the yield of cyclohexanone was 26.3 %, the yield of cyclohexanol was 37.1 %, and the yield of cyclohexyl hydroperoxide was 17.3 %.

[Comparative Example 3-6]

The oxidation of cyclohexane was carried out in the same manner as in Example 3-14 except that SUS316L column type reactors without glass lining were used as the first, second, and third columns. The result showed that the conversion of cyclohexane was 5.3 %, the yield of cyclohexanone was 26.4 %, the yield of cyclohexanol was 38.0 %, and the yield of cyclohexyl hydroperoxide was 13.3 %.

[Comparative Example 3-7]

The oxidation of cyclohexane was carried out in the same manner as in Example 3-14 except that the reaction temperature of the first column decreased to 155°C, that the further addition of cyclohexane to the second and third column was not carried out, and that the speed of injecting air to the first, second, and third column was changed to 45 NL/h, 38 NL/h, and 38 NL/h, respectively. In this case, the reaction temperature of the second column was increased to 160.4°C, and the reaction temperature of the third column was increased to 165.5°C. The result showed that the conversion of cyclohexane was 5.2 %, the yield of cyclohexanone was 23.0 %, the yield of cyclohexanol was 47.5 %, and the yield of cyclohexyl hydroperoxide was 7.1 %.

[Comparative Example 3-8]

As shown in FIG. 2, an oxidation reaction solution in an oxidation reactor 40 was transferred to an experimental apparatus 50 for decomposing hydroperoxide separated 50 meters away from the oxidation reactor 40 at a flow rate of 900 L/h. The piping connected between the oxidation reactor 40 and the experimental apparatus 50 for decomposing hydroperoxide was made of SUS316, and the inner diameter of the lining was 3/4 inches. A transfer pump 41 was mounted in the middle of the piping. When an oxidation reaction solution before transferring was taken from a sampling nozzle 42, the solution had a temperature of 150°C, and contained 0.82 % by weight of cyclohexyl hydroperoxide. On the other hand, an aqueous alkaline solution tank 45 was connected to the inlet of the experimental apparatus 50 for decomposing hydroperoxide. In an oxidation reaction solution taken from a sampling nozzle 43 in the upstream of the aqueous alkaline solution tank 45, the concentration of cyclohexyl hydroperoxide was 0.31 % by weight. Therefore, 62.2 % of cyclohexyl hydroperoxide contained in the oxidation reaction solution was decomposed in the piping of the transferring unit.

[Example 3-16]

The oxidation of cyclohexane was carried out in the same manner as in Comparative Example 8 except that the inner part of the transfer pump was coated with a Teflon (registered trademark), and the piping was replaced by a Teflon (registered trademark) inner tube having an inner diameter of 3/4 inches. The result showed that the concentration of cyclohexyl hydroperoxide in an oxidation reaction solution taken just before the inlet of the experimental apparatus for decomposing hydroperoxide was 0.68 % by weight, and the decomposition rate in the transferring unit was 17.1 %.

## Claims

1. A method for oxidizing a linear or cyclic saturated hydrocarbon compound with molecular oxygen to produce a ketone and/or an alcohol, comprising:

   (a) oxidizing the a linear or cyclic saturated hydrocarbon compound at a temperature of 120°C or more and 160°C or less using a serial multistage reacting unit to obtain the corresponding hydroperoxide;
   (b) decomposing the hydroperoxide by adding an aqueous alkaline solution to obtain the corresponding ketone and alcohol; and
   (c) transferring the reaction solution obtained by the step (a) to a unit in which the step (b) is carried out;

   wherein a reacting unit for carrying out at least the final stage reaction in the step (a) and a transferring unit for the step (c) have an inner surface formed by a material from which no transition metal ion is generated.

2. The method according to claim 1, wherein the material, from which no transition metal ion is generated, is a fluorine resin and/or a glass.

3. The method according to claim 1 or 2, wherein a reacting unit(s) in the downstream of the first reactor of the serial multistage reacting unit and the transferring unit have an inner surface formed by the material from which no transition metal ion is generated.

4. The method according to any one of claims 1 to 3, wherein the temperature of the step (a) is 155°C or less.

5. The method according to any one of claims 1 to 4, wherein an N-hydroxy compound represented by any one of the following general formula (3), general formula (5a), or general formula (5b) is added as an oxidation catalyst to the reaction solution for the step (a):

   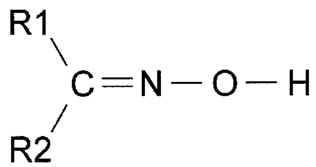 (3)

   wherein, in the general formula (3), R1 and R2 are each independently a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, a substituted or unsubstituted aryl group, a substituted or unsubstituted aralkyl group, a substituted or unsubstituted heterocyclic group, or hydrogen atom; or R1 and R2 bind to each other to be a divalent organic group forming a nonconjugated ring;

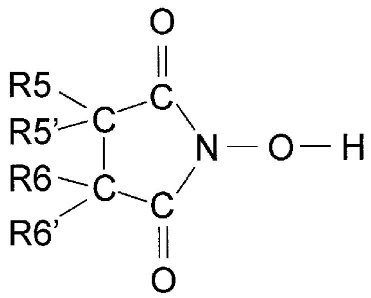

(5a)

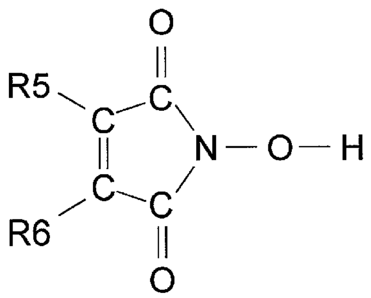

(5b)

wherein, in the general formulae (5a) and (5b), R5, R5', R6, and R6' are each independently hydrogen atom, a halogen atom, a substituted or unsubstituted alkyl group, a substituted or unsubstituted aryl group, a substituted or unsubstituted cycloalkyl group, a hydroxyl group, a substituted or unsubstituted alkoxy group, a carboxyl group, a substituted or unsubstituted alkoxycarbonyl group, or a substituted or unsubstituted acyl group; or R5 or R5' and R6 or R6' bind to each other to be a divalent organic group forming a ring.

6. The method according to any one of claims 1 to 5, wherein the hydrocarbon compound is cyclohexane, the ketone is cyclohexanone, and the alcohol is cyclohexanol.

**Patentansprüche**

1. Verfahren zum Oxidieren einer linearen oder cyclischen gesättigten Kohlenwasserstoffverbindung mit molekularem Sauerstoff, um ein Keton und/oder einen Alkohol herzustellen, umfassend:

(a) Oxidieren der linearen oder cyclischen gesättigten Kohlenwasserstoffverbindung bei einer Temperatur von 120 °C oder mehr und 160 °C oder weniger unter Verwendung einer seriellen Mehrstufen-Umsetzungseinheit, um das entsprechende Hydroperoxid zu erhalten;
(b) Zersetzen des Hydroperoxids durch Hinzufügen einer wässrigen alkalischen Lösung, um das entsprechende Keton und den entsprechenden Alkohol zu erhalten; und
(c) Übertragen der durch den Schritt (a) erhaltenen Reaktionslösung an eine Einheit, in der der Schritt (b) ausgeführt wird;

wobei eine Umsetzungseinheit zum Ausführen zumindest der letzten Reaktionsstufe in dem Schritt (a) und eine Übertragungseinheit für den Schritt (c) eine innere Oberfläche aufweisen, die durch ein Material gebildet ist, aus dem kein Übergangsmetallion erzeugt wird.

2. Verfahren nach Anspruch 1, wobei das Material, aus dem kein Übergangsmetallion erzeugt wird, ein Fluorharz und/oder ein Glas ist.

3. Verfahren nach Anspruch 1 oder 2, wobei eine Umsetzungseinheit, die stromabwärts des ersten Reaktors der seriellen Mehrstufen-Umsetzungseinheit und der Übertragungseinheit liegt, eine innere Oberfläche aufweist, die durch das Material gebildet ist, aus dem kein Übergangsmetallion erzeugt wird.

4. Verfahren nach einem der Ansprüche 1 bis 3, wobei die Temperatur des Schrittes (a) 155 °C oder weniger beträgt.

**5.** Verfahren nach einem der Ansprüche 1 bis 4, wobei eine N-Hydroxyverbindung, die durch eine beliebige der folgenden allgemeinen Formel (3), allgemeinen Formel (5a) oder allgemeinen Formel (5b) dargestellt ist, als ein Oxidationskatalysator zu der Reaktionslösung für den Schritt (a) hinzugefügt wird:

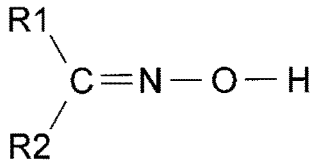

(3)

wobei, in der allgemeinen Formel (3), R1 und R2 jeweils unabhängig eine substituierte oder unsubstituierte Alkylgruppe, eine substituierte oder unsubstituierte Alkenylgruppe, eine substituierte oder unsubstituierte Alkynylgruppe, eine substituierte oder unsubstituierte Cycloalkylgruppe, eine substituierte oder unsubstituierte Cycloalkenylgruppe, eine substituierte oder unsubstituierte Arylgruppe, eine substituierte oder unsubstituierte Aralkylgruppe, eine substituierte oder unsubstituierte heterocyclische Gruppe oder ein Wasserstoffatom sind; oder R1 and R2 jeweils aneinander binden, um eine divalente organische Gruppe zu sein, die einen nichtkonjugierten Ring bildet;

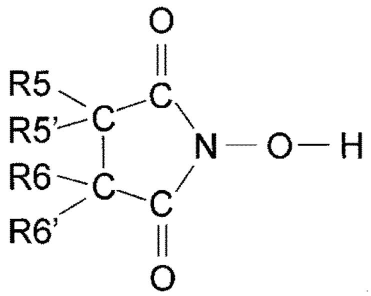

(5a)

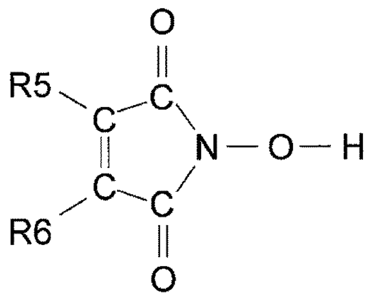

(5b)

wobei, in den allgemeinen Formeln (5a) und (5b), R5, R5', R6 und R6' jeweils unabhängig ein Wasserstoffatom, ein Halogenatom, eine substituierte oder unsubstituierte Alkylgruppe, eine substituierte oder unsubstituierte Arylgruppe, eine substituierte oder unsubstituierte Cycloalkylgruppe, eine Hydroxylgruppe, eine substituierte oder unsubstituierte Alkoxygruppe, eine Carboxylgruppe, eine substituierte oder unsubstituierte Alkoxycarbonylgruppe oder eine substituierte oder unsubstituierte Acylgruppe sind; oder R5 oder R5' und R6 oder R6' jeweils aneinander binden, um eine divalente organische Gruppe zu sein, die einen Ring bildet.

**6.** Verfahren nach einem der Ansprüche 1 bis 5, wobei die Kohlenwasserstoffverbindung Cyclohexan ist, das Keton Cyclohexanon ist und der Alkohol Cyclohexanol ist.

## Revendications

**1.** Procédé d'oxydation d'un composé hydrocarboné saturé linéaire ou cyclique avec de l'oxygène moléculaire afin de produire une cétone et/ou un alcool, comprenant :

(a) l'oxydation du composé hydrocarboné saturé linéaire ou cyclique à une température supérieure ou égale à 120 °C et inférieure ou égale à 160 °C au moyen d'une unité de réaction multi-étape en série afin d'obtenir

l'hydroperoxide correspondant ;

(b) la décomposition de l'hydroperoxide par l'ajout d'une solution alcaline aqueuse afin d'obtenir la cétone et l'alcool correspondants ; et

(c) le transfert de la solution de réaction obtenue lors de l'étape (a) vers une unité dans laquelle l'étape (b) est effectuée ;

dans lequel une unité de réaction pour la réalisation d'au moins la réaction de l'étape finale dans l'étape (a) et une unité de transfert pour l'étape (c) ont une surface intérieure formée par un matériau à partir duquel aucun ion de métal de transition n'est généré.

**2.** Procédé selon la revendication 1, dans lequel le matériau, à partir duquel aucun ion de métal de transition n'est généré, est une résine de fluor et/ou un verre.

**3.** Procédé selon la revendication 1 ou la revendication 2, dans lequel au moins une unité de réaction en aval du premier réacteur de l'unité de réaction multi-étape en série et l'unité de transfert ont une surface interne formée par le matériau à partir duquel aucun ion de métal de transition n'est généré.

**4.** Procédé selon l'une quelconque des revendications 1 à 3, dans lequel la température de l'étape (a) est inférieure ou égale à 155 °C.

**5.** Procédé selon l'une quelconque des revendications 1 à 4, dans lequel un composé N-hydroxy représenté par l'une quelconque parmi la formule générale (3), la formule générale (5a), ou la formule générale (5b) suivante est ajouté en tant que catalyseur d'oxydation à la solution de réaction pour l'étape (a) :

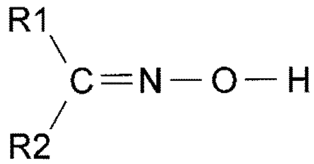

(3)

dans lequel, dans la formule générale (3), R1 et R2 représentent chacun indépendamment un groupe alkyle substitué ou non substitué, un groupe alcényle substitué ou non substitué, un groupe alcynyle substitué ou non substitué, un groupe cycloalkyle substitué ou non substitué, un groupe cycloalcényle substitué non substitué, un groupe aryle substitué ou non substitué, un groupe aralkyle substitué ou non substitué, un groupe hétérocyclique substitué ou non substitué, ou l'atome d'hydrogène ; ou R1 et R2 se lient les uns aux autres pour former un groupe organique divalent formant un cycle non conjugué ;

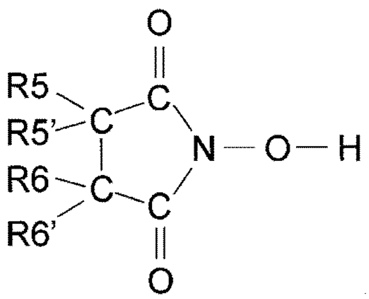

(5a)

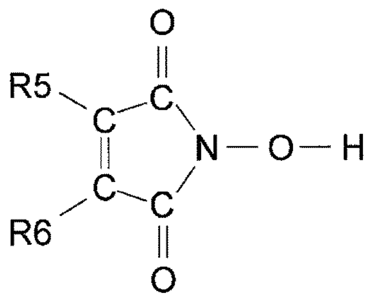

(5b)

dans lequel, dans les formules générales (5a) et (5b), R5, R5', R6, et R6' représentent chacun indépendamment l'atome d'hydrogène, un atome d'halogène, un groupe alkyle substitué ou non substitué, un groupe aryle substitué ou non substitué, un groupe cycloalkyle substitué ou non substitué, un groupe hydroxyle, un groupe alcoxy substitué ou non substitué, un groupe carboxyle, un groupe alcoxycarbonyle substitué ou non substitué, ou un groupe acyle substitué ou non substitué ; ou R5 ou R5' et R6 ou R6' se lient les uns aux autres pour former un groupe organique divalent formant un cycle.

**6.** Procédé selon l'une quelconque des revendications 1 à 5, dans lequel le composé hydrocarboné est le cyclohexane, la cétone est la cyclohexanone, et l'alcool est le cyclohexanol.

FIG. 1

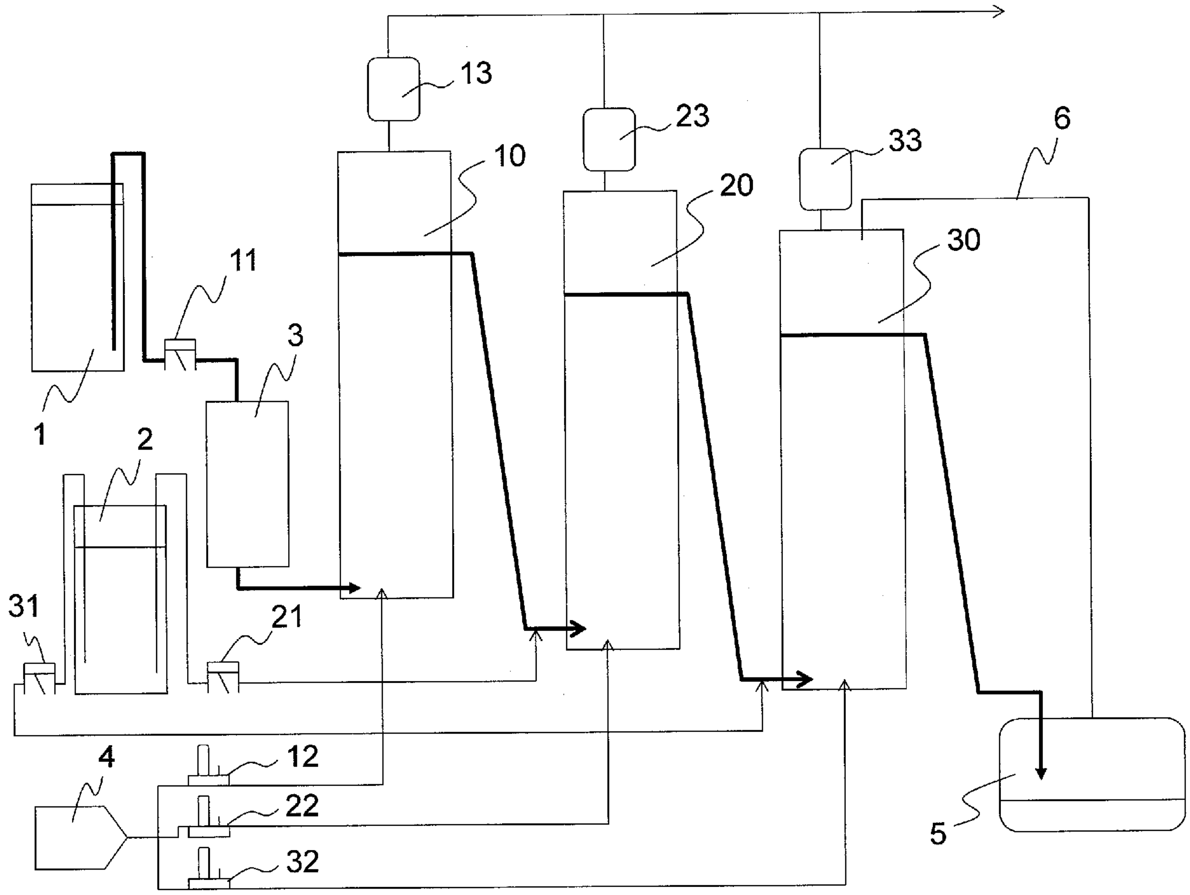

FIG. 2

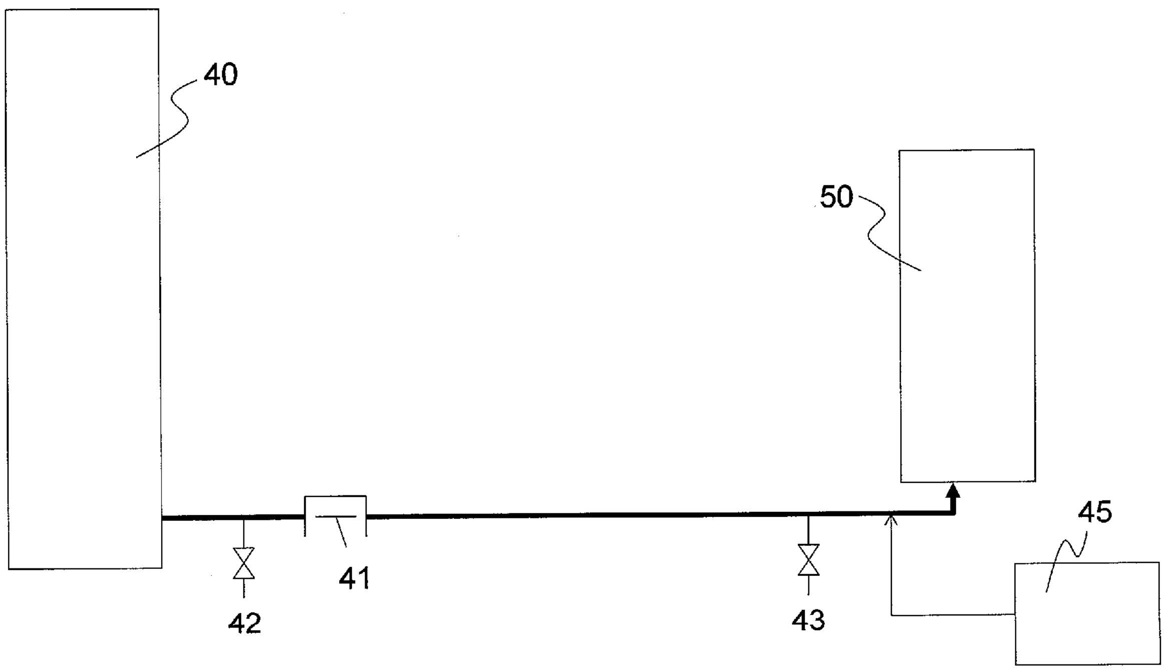

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 62120359 A
- JP 8038909 A
- JP 2011054809 A
- JP 2007510724 A
- WO 2009025939 A


### Non-patent literature cited in the description


- **J. A. KERR.** *Chem. Rev.,* 1966, vol. 66, 465
- *J. Org. Chem.,* 1997, vol. 62, 6810
- *J. Am. Chem. Soc.,* 1984, vol. 106, 3374
- **YOSHIO KAMIYA.** Organic Oxidation Reaction (Yuki Kagaku Hannou. GIHODO, 05 August 1973